# EUROPEAN PATENT APPLICATION

(11) **EP 4 057 006 A1**
(43) Date of publication of application: **14.09.2022**
(21) Application number: 21305275.6
(22) Date of filing: 08.03.2021
(51) Int. Cl.: G01N 33/574, G01N 33/68

(54) **EX VIVO METHOD FOR ANALYSING A TISSUE SAMPLE USING PROTEOMIC PROFILE MATCHING, AND ITS USE FOR THE DIAGNOSIS, PROGNOSIS OF PATHOLOGIES AND FOR PREDICTING RESPONSE TO TREATMENTS**

(71) Applicant: UNIVERSITE DE BORDEAUX, 33000 Bordeaux (FR); INSTITUT NATIONAL DE LA SANTE ET DE LA RECHERCHE MEDICALE - INSERM, 75013 Paris (FR); Centre Hospitalier Universitaire de Bordeaux, 33404 Talence Cedex (FR)
(72) Inventor: SALTEL, Frédéric, 33400 TALENCE (FR); RAYMOND, Anne-Aurélie, 33700 MERIGNAC (FR); Di TOMMASO, Sylvaine, 33600 PESSAC (FR); DOURTHE, Cyril, 33700 MERIGNAC (FR)
(74) Representative: Novagraaf Technologies

(57) **Abstract**

The present invention relates to a computer-implemented method for determining a reference proteomic profile of a pathological condition in a subject, comprising steps of :
- label free quantification of the relative protein abundances of a tissue sample from at least one subject for which a diagnosis of a pathological condition of said tissue has been previously established, said sample containing non-pathological and pathological tissue,
- determining the proteomic profile of said sample by calculating the ratio of the relative abundances, for each protein, of said protein in the pathological tissue with respect to said protein in the non-pathological tissue,
- determining a reference proteomic profile for the previously diagnosed pathological condition, by means of a statistical test established between at least two groups of subjects.

The invention also relates to a data processing device comprising means for carrying out the method of the invention, to an *ex vivo* method for analysing a tissue likely to be pathological in a subject, to an *ex vivo* method of proteomic profiling of a tissue, to an *ex vivo* method for assessing a risk of transformation of a benign tumour to cancer in a subject, to an *ex vivo* method for the differential diagnosis of benign and malignant tumours in a subject, and to an *ex vivo* method for predicting the efficacy of a treatment for a pathology in a subject.

## Description

### Technical field

The present invention refers to a computer-implemented method for determining a reference proteomic profile of a pathological condition in a subject, and to its use in the *ex vivo* analysis of tissue samples, especially for diagnosis and management of a condition in a subject.

Therefore, the present invention has utility in medical field, and more particularly in diagnosis, prognosis and theranostic fields.

In the description below, the references into brackets ([ ]) refer to the listing of references situated at the end of the text.

### Background of the Invention

Pathology diagnosis and management, especially for a tumor, require different clinical expertise. Today, medical imaging has made enormous progress and provides effective results in terms of diagnosis and tumor prognosis. However, one of the major challenges often remains to determine whether a tumor is malignant or not. If it is benign, it's important to know whether it still presents clinical risks, including malignant transformation into cancer. If a cancer is identified, the maximum of biological information must be obtained in order to propose the best possible patient management and more precisely, a therapeutic approach adapted the tumor.

Biopsy is an invasive procedure that is generally the first unavoidable step required to establish the diagnosis. The histological analysis of biopsies is an essential tool for diagnosis complementary to medical imaging. Pathologists can analyze the morphological features of tissues and visualize biomarker expression by immunohistochemistry (IHC) to determine the nature of tumor. Today, various biomarkers are currently available to support diagnosis and prognosis, and theranostics and pathologists use serial immunostainings to investigate and evaluate the abundance of an increasing number of biomarkers. Other molecular approaches, such as PCR (Polymerase Chain Reaction) and FISH (Fluorescence in situ hybridization), are routinely performed on human samples. Recent technological advances now make it possible to carry out very advanced genomic (next generation sequencing) and transcriptomic (RNA sequencing) analyses from frozen and even formalin-fixed and paraffin-embedded (FFPE) tissues.

Nevertheless, the latter are not completely suited to such analyses given the degradation of genetic material. These molecular analyses can be used for mutation analysis or for detecting the presence of certain transcripts in routine care samples. However, in addition to the IHC analyses performed, they often exhaust the biological sample. Downstream of translation, proteomic analysis allows the extraction of a considerable amount of information on the functional status of a disease, directly identifying and quantifying potential biomarker proteins (Sala M, et al.: "ASS1 Overexpression: A Hallmark of Sonic Hedgehog Hepatocellular Adenomas". Recommendations for Clinical Practice. Hepatol. Commun. 4, 809-824 (2020) ([1])) and pharmacological targets usable in clinical practice (Gustafsson O, et al. "P. Proteomic developments in the analysis of formalin-fixed tissue". Biochim. Biophys. Acta BBA - Proteins Proteomics 1854, 559-580 (2015) ([2])). However, to date, proteomics is not effective in diagnosing benign versus malignant tumors.

In-depth analysis of FFPE tissue proteomes is a technique now commonly used in translational research (Coscia F, et al. "A streamlined mass spectrometry-based proteomics workflow for largescale FFPE tissue analysis". J. Pathol. 251, 100-112 (2020) ([3]); Henriet E, et al. "Argininosuccinate synthase 1 (ASS1): A marker of unclassified hepatocellular adenoma and high bleeding risk". Hepatol. Baltim. Md 66, 2016-2028 (2017) ([4])). FFPE tissue proteomics with laser microdissection have already been combined to enable the precise selection of a tissue area of interest, and this technique was optimized for compatibility with very small amounts of material, such as needle microbiopsies (Sala M, et al. ([1])). Indeed, a 1 mm² area of a 5 µm thick FFPE section is sufficient to compare deregulations in protein expression of 1000 proteins in pathological versus healthy tissues. Previously, using this method in an exploratory approach, a new diagnostic biomarker for a distinctive subtype of hepatocellular adenomas has been identified (HCA) (Sala M, et al. ([1]); Henriet E, et al. ([4])). It has also been shown that when in doubt of IHC staining interpretation, especially on small liver biopsies, HCA biomarker quantification by mass spectrometry could in fact be a valuable complement of information in diagnosis support (Sala M, et al. ([1])).

Experts in histoproteomics agree that proteomic analysis will integrate the surgical pathologist field in the near future (Coscia F, et al. ([3]); Ahmed M, et al. "Next-generation protein analysis in the pathology department". J. Clin. Pathol. 73, 1-6 (2020) ([5]); Jin P, et al. "Pathology, proteomics and the pathway to personalised medicine". Expert Rev. Proteomics. 15, 231-243 (2018) ([6])). Mass spectrometry already meets some specific requirements for biomarker identification. However, experts often agree that a single biomarker is never 100% specific and can't be sufficient for diagnosic, prognosic or theranostic approaches.

Thus, a need exists of alternative diagnostic tools, allowing patient management and predicting response to treatments to improve their efficiency. The present invention fulfills these and other needs.

### Description of the invention

The Applicant has found that a profile of protein expression deregulation in a tissue, especially the whole profile of protein expression deregulation, could be diagnostic and management of a pathological entity in a much more complete way than a single biomarker.

The Applicant carried out pioneering work using proteomic pattern matching for diagnosis from tissues. After extensive research, the Applicant developed proteomic profiling to improve diagnosis and therefore patient management.

The Applicant developed an inventive tool based on the comparison of a proteomic profile extracted from a biopsy or surgical specimen with a reference database composed of proteomic profiles from completely characterized patients.

The extensive work of the Applicant leads to an analytical process for operational routine analysis. Furthermore, only small amounts of tissue are needed for this analysis, thus not depleting the samples and it can be reused. Another impressive advantage of the process is that the complete analysis can be achieved within a week and so it remains compatible with the deadlines imposed by clinical practice. Moreover, the proteomic data generated remains a resource that can be re-exploited for further applications (prognosis, theranostics).

The Applicant demonstrated that a main advantage of working on a profile of protein deregulation (pathological vs non-pathological) is the ability to directly access the pathological tissue identity by reducing inter-individual variation, making the analysis efficient despite small patient collections. This also allows the association of proteomic profile matchings with functional biological pathways, the control of biological relevance, and identification of new targets. An additional advantage of working on a profile of protein expression deregulation versus spectral intensity profiles is that the construction of the reference database is not dependent on mass spectrometer type. Indeed, the method of the invention can be implemented on sites with different equipment, contrary to radiomics that encounter major obstacles concerning the heterogeneity between devices used for image acquisition.

Moreover, the Applicant developed proteomic-based machine learning analysis that is operational in routine clinical practice and could provide progression in patient diagnosis.

Accordingly, in a first aspect, the present invention provides a computer-implemented method for determining a reference proteomic profile of a pathological condition, comprising the steps of :
- label free quantification of the relative protein abundances of a tissue sample from at least one subject for which a diagnosis of a pathological condition of said tissue has been previously established, said sample containing non-pathological and pathological tissue,
- determining the proteomic profile of said sample by calculating the ratio of the relative abundances, for each protein, of said protein in the pathological tissue with respect to said protein in the non-pathological tissue,
- determining a reference proteomic profile for the previously diagnosed pathological condition, by means of a statistical test established between at least two groups of subjects.

"Reference proteomic profile" refers herein to a profile of protein expression established for a diagnosed pathological condition, which can be used as a comparative reference for analyzing non-diagnosed tissues.

According to the invention, "pathological condition » refers herein to any disease, physical condition resulting from a disease, or physical condition likely to develop into a disease. It may be for example a cancer, an autoimmune disease, a thrombosis, an inflammatory disease, an infection, a graft or prosthesis rejection, or a benign tumor, this being not limitative.

The method of the invention may be realized on tissue samples that may be of any kind likely to be analyzed by mass spectrometry. It may be obtained by any method known in the state of the art, for example by macrodissection or laser microdissection of a formalin-fixed paraffin-embedded or frozen tissue. The steps of preparation of histological sections, protein extraction, preparation of samples for mass spectrometry analysis, and laser microdissection cutting, formalin fixation reversal and freezing if any, may be realized according to methods known in the art, for example as described by Sala M, et al. ([1]), and Henriet E, et al. ([2]).

The tissue may be any kind of human or animal tissue commonly used in the state of the art for tissue analysis, for example breast, liver, kidney, lung, uterus, prostate, testis, tongue, skin, bone marrow, colon, bronchi, and/or muscle tissue, pancreas, endocrine tissues, brain, eye, urinary bladder, gallbladder, salivary glands or lymphoid tissues.

As mentioned above, a tissue sample used in the method of the invention contains both non-pathological and pathological tissue. « Pathological tissue » refers herein to a tissue area affected by the pathological condition previously established, whereas « non-pathological tissue » refers to a tissue area not affected by the pathological condition. The identification of the pathological area of a tissue or the non-pathological area of a tissue may be realized by any method known in the art, for example by visual identification of morphological features of the tissue or identified by immunohistological analysis on a serial section.

In the method for determining a reference proteomic profile of a pathological condition, the tissue sample may be obtained from at least one subject. Advantageously, the tissue samples may be obtained from at least 5, or at least 10, or at least 15, or at least 20, or at least 30, or at least 40, or at least 50, or at least 60, or at least 70, or at least 100, or at least 150, or at least 200, or more subjects. Advantageously, the more subjects there are, the more "robust" the profile is.

"Label free quantification" refers herein to a method in mass spectrometry allowing to extract and compare the relative abundance of proteins in at least two tissue samples, without using a stable isotope containing compound labelling the proteins. The method may be any method known in the state of the art, for example as described by Sala M, et al. ([1]), Henriet E, et al. ([2]). The quantitative data may be analyzed using any data analytics software commonly used for this purpose in the state of the art, as for example the R software. As commonly realized in this technical field, replicates may be realized for each subject, regarding pathological tissue and non-pathological tissue.

According to the invention, the relative protein abundances may be determined for the whole proteins identified in the tissue sample, or for part of these proteins. According to the invention, the relative protein abundances are compared between non-pathological and on pathological areas of the tissue sample.

A pathological tissue/ non-pathological tissue ratio may be calculated for each protein, or from technical replicate medians if any. Advantageously, proteomic profiles from characterized subjects may compose a reference database.

"Groups of subjects" refers herein to groups or subjects that are to be compared through their proteomic profiles. Advantageously, it may correspond to comparing proteomic profile of the above-mentioned sample with the one of at least another group of subjects or at least another subjects. For example, it may be used for :
- comparing healthy and pathological tissue,
- comparing two groups of patients with good and poor responders to treatment,
- comparing patients who are good responders having two or three treatments (this also allows for comparison of several groups),
- comparing a group that has relapsed vs. a group that has not relapsed, and
- comparing a group that has developed a mail form vs. a group that has not done so.

Statistical analysis of the ratios thus obtained may be realized by any method known for similar purpose in the state of the art. It may be for example a Wilcoxon-Mann-Whitney U-test or a t-test. The skilled person is able to choose one or more of statistical analysis regarding his knowledge of this technical field.

Advantageously, the computer-implemented method of the invention involves the use of tools commonly used in this technical field, as for example, a record component, notably for recording, for each sample, the proteomic profile in relation to the previously established pathological condition diagnosis, a storage component for storing data, a computer processor for processing data, wherein the computer processor is coupled to the storage component and configured to execute the instructions stored in the storage component in order to receive data and analyze them according to one or more algorithms; and a display component for displaying information regarding the reference proteomic profile.

Hence, another object of the invention relates to a data processing device comprising means for carrying out the computer-implemented method for determining a reference proteomic profile of a pathological condition as defined above.

The invention also relates to several methods that shares some steps with the above-described method, and are based on the general inventive concept described above. Therefore, for the following description of the invention, the same terms have the same meaning, *mutatis mutandis,* than given above.

Thus, another object of the invention relates to an *ex vivo* method of proteomic profiling of a tissue, comprising the following steps:
- label-free quantification of the relative protein abundances of a sample of said tissue, said sample containing non-pathological tissue and tissue likely to be pathological, and
- determining the proteomic profile of said tissue, by calculating the ratio, for each protein, of the relative amount of said protein in the tissue likely to be pathological with respect to the amount of said protein in the non-pathological tissue.

Another object of the invention relates to an *ex vivo* method for analyzing a tissue likely to be pathological, in a subject, comprising the following steps:
- label-free quantification of the relative protein abundances of a tissue sample from said subject, said sample containing non-pathological tissue and tissue likely to be pathological,
- determining the proteomic profile of said sample, by calculating the ratio of the relative abundances, for each protein, of the relative amount of said protein in the tissue likely to be pathological with respect to the amount of said protein in the non-pathological tissue,
- calculating the similarity score between the proteomic profile of said sample and at least one reference proteomic profile obtained by the computer-implemented method for determining a reference proteomic profile of a pathological condition as defined above.

Advantageously, the similarity score may be calculated by any known method commonly used by the skilled person in this technical field. It may be any method for calculations of similarity, for example Chi-square test on simplified data, Euclidean distance calculation after PCA or Random Forest on data reduced by PCA.

As already explained above, the *ex vivo* method for analyzing of the invention is based on the analysis of the profile of protein expression deregulation in said tissue likely to be pathological with respect to a reference profile, *i.e.* by proteomic profile matching.

Another object of the invention relates to an *ex vivo* method of diagnosing a pathology in a subject, comprising carrying out the *ex vivo* method of analysis as defined above, wherein a similarity score between the proteomic profile of the sample from said subject and the reference proteomic profile of the pathology is indicative of said pathology.

Advantageously, the *ex vivo* method of diagnosing of the invention is based on proteomic pattern matching, and can be realized during clinical routine practice. This method of diagnosing may comprise the phases commonly carried out in this technological field, as :
(i) an examination phase, involving the collection of data. Advantageously, this step may include at least the label-free quantification of the relative protein abundances of a tissue sample from said subject, and the determination of the proteomic profile of said sample, as defined above,
(ii) the comparison of these data with standard values. This step may include at least calculating the similarity score between the proteomic profile of said sample and at least one reference proteomic profile as defined above,
(iii) the finding of any significant deviation, *i.e*. a symptom, during the comparison,
(iv) the attribution of the deviation to a particular clinical picture, i.e. the deductive medical decision phase (diagnosis for curative purposes stricto sensu).

Another object of the invention relates to an *ex vivo* method for assessing a risk of transformation of a benign tumor into cancer in a subject, comprising carrying out the *ex vivo* method of analysis of a tissue as defined above, wherein a similarity score of the proteomic profile of the sample of said subject with the reference proteomic profile of a cancer is indicative of a risk of transformation of said benign tumour into cancer.

According to the invention, the benign tumour may be any tumor at risk to transform into a cancer. It can be for example a hepatocellular adenoma, which can develop into a hepatocellular cancer, in particular a hepatocellular carcinoma. This method of for assessing a risk of transformation of a benign tumor into cancer may comprise the phases commonly carried out in this technological field, as :
(i) an examination phase, involving the collection of data. Advantageously, this step may include at least the label-free quantification of the relative protein abundances of a tissue sample from said subject, and the determination of the proteomic profile of said sample, as defined above,
(ii) the comparison of these data with standard values. This step may include at least calculating the similarity score between the proteomic profile of said sample and at least one reference proteomic profile of a cancer,
(iii) the finding of any significant deviation, i.e. a symptom, during the comparison,
(iv) the attribution of the deviation to a clinical picture of risk of transformation of a benign tumor into cancer.

Another object of the invention relates to an *ex vivo* method for the differential diagnosis of benign and malignant tumours in a subject, comprising carrying out the *ex vivo* diagnostic method as defined above, wherein a similarity score of the proteomic profile of the sample from said subject with the reference proteomic profile of a tumour is indicative of said tumour, and optionally its subtype and malignant transformation state.

This method for the differential diagnosis of benign and malignant tumours may comprise the phases commonly carried out in this technological field, as :
(i) an examination phase, involving the collection of data. Advantageously, this step may include at least the label-free quantification of the relative protein abundances of a tissue sample from said subject, and the determination of the proteomic profile of said sample, as defined above,
(ii) the comparison of these data with standard values. This step may include at least calculating the similarity score between the proteomic profile of said sample and at least one reference proteomic profile of a cancer or of a subtype of benign or malignant tumor,
(iii) the finding of any significant deviation, i.e. a symptom, during the comparison,
(iv) the attribution of the deviation to a clinical picture of a cancer or of a subtype of benign or malignant tumor.

As mentioned above, the tumor may be any tumor, for example a liver tumor. It may be more particularly a hepatocellular carcinoma, and the benign tumor may be a hepatocellular adenoma. Advantageously, the hepatocellular adenoma may belong to at least one subtype selected from H-HCA, IHCA, b-HCA, b-IHCA and sh-HCA.

Another object of the invention relates to an *ex vivo* method for identifying at least one biomarker of a pathology, comprising carrying out the computer-implemented method.

Another object of the invention relates to an *ex vivo* method for predicting the efficacy of a treatment for a pathology in a subject, comprising carrying out the *ex vivo* diagnostic method as defined above, wherein a similarity score between the proteomic profile of the sample from said subject and the reference proteomic profile of the pathology is predictive of a response or non-response to the treatment for said pathology.

Advantageously the subject has a malignant tumor, for example a liver tumor. Advantageously the pathology is a liver cancer, in particular a hepatocellular carcinoma (HCC). Advantageously the treatment for HCC is a sorafenib treatment.

In particular the invention relates to an *ex vivo* method for predicting the efficacy of a treatment for a liver cancer in a subject, comprising carrying out the *ex vivo* diagnostic method as defined above, wherein a similarity score between the proteomic profile of the sample from said subject and the reference proteomic profile of a hepatocellular carinoma is predictive of a response to the treatment for said hepatocellular carcinoma.

In particular the invention relates to an *ex vivo* method for predicting the efficacy of a treatment for a pathology in a subject, comprising carrying out the *ex vivo* diagnostic method as defined above, wherein a similarity score between the proteomic profile of the sample from said subject and the reference proteomic profile of the pathology is predictive of a non-response to the treatment for said pathology. The pathology may be any kind of pathology, for example cancers, especially hepatocellular carinomas.

This invention is further illustrated by the following examples with regard to the annexed drawings that should not be construed as limiting.

### Brief description of the figures

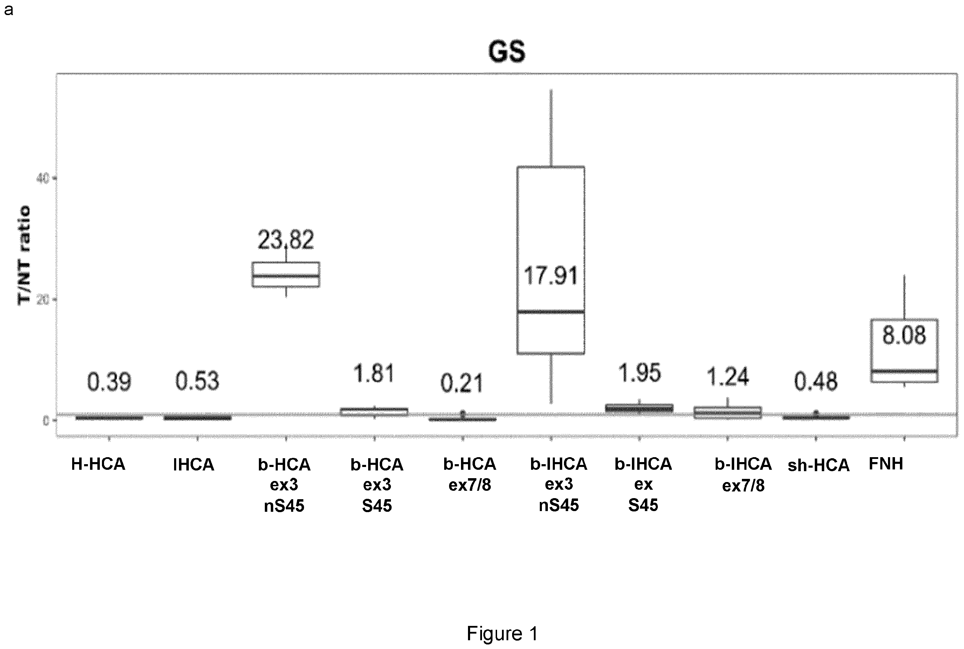

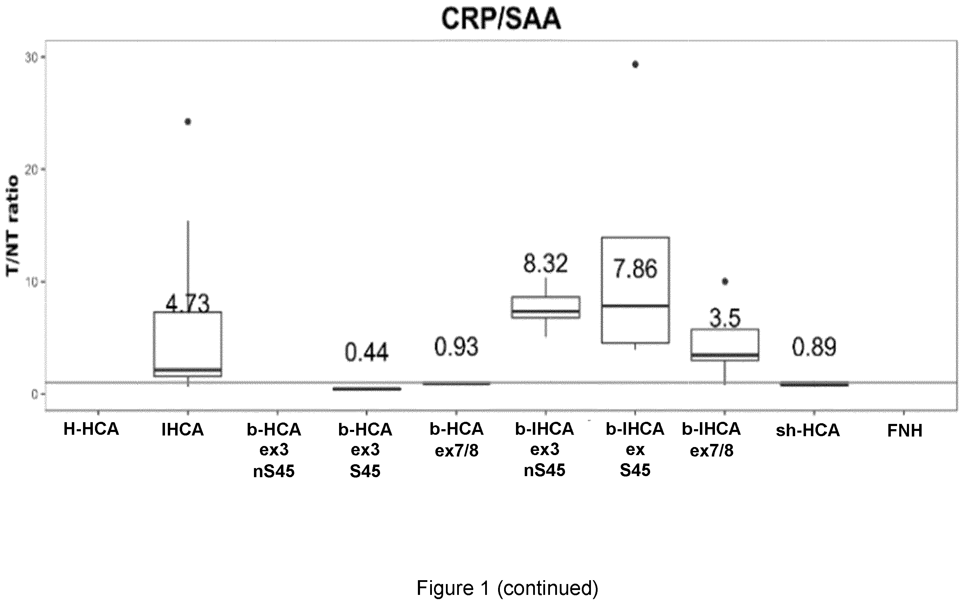

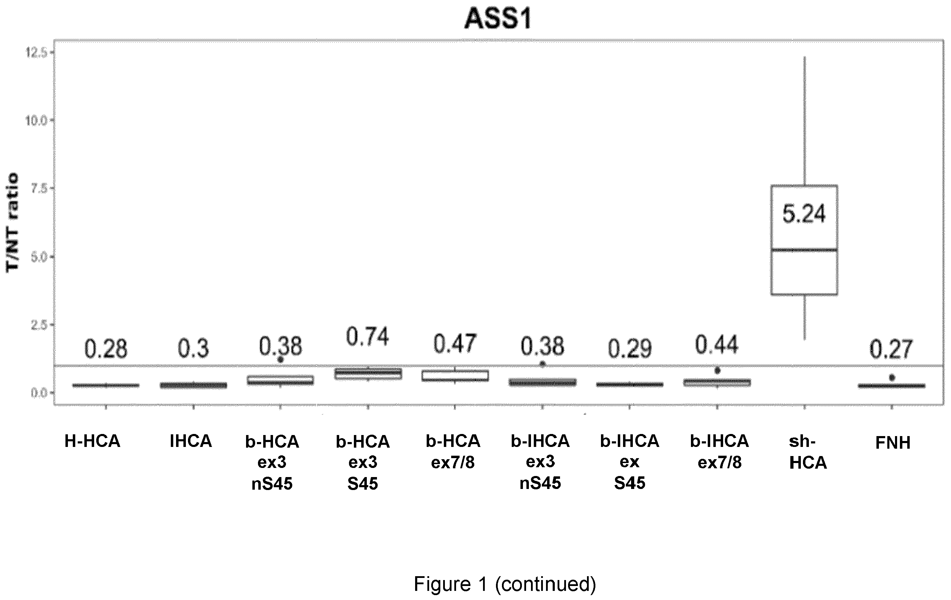

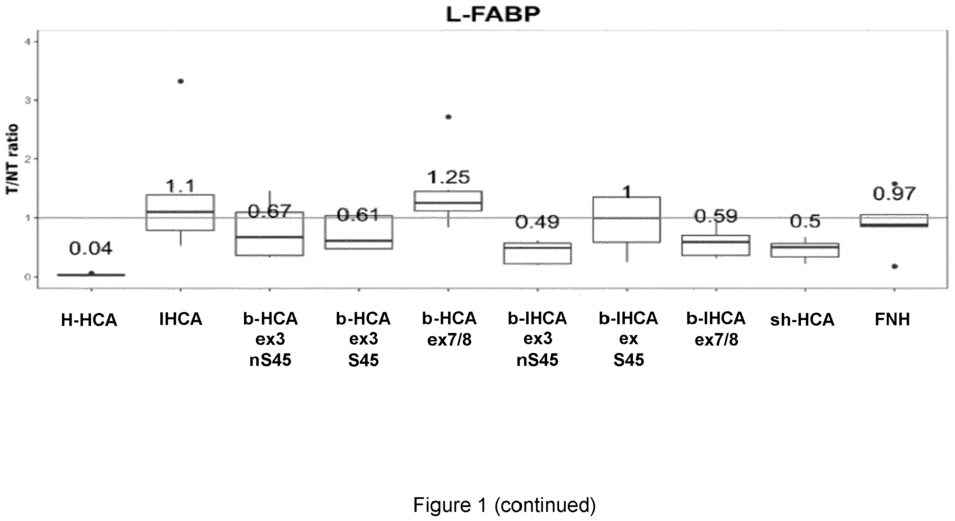

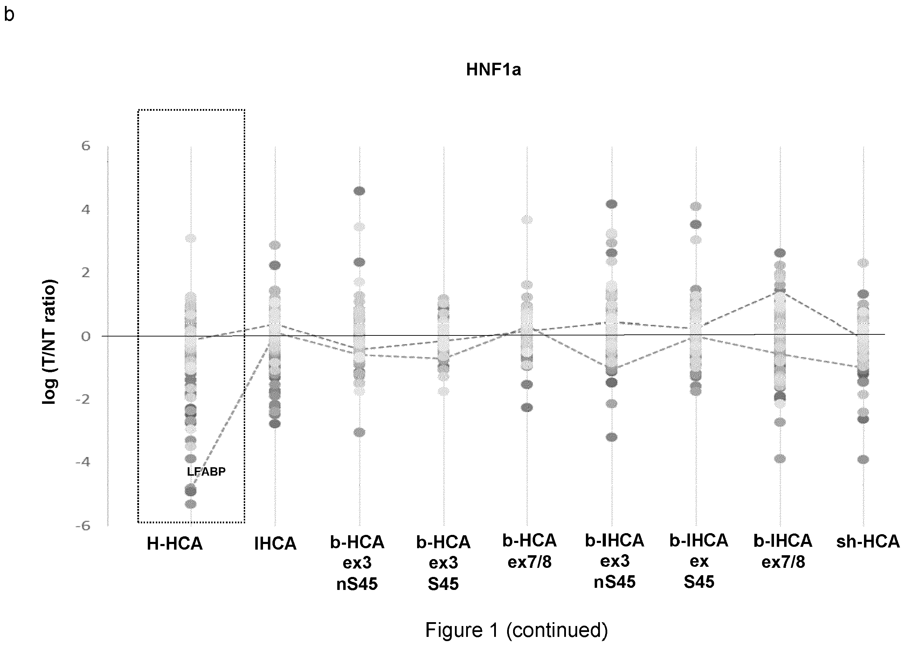

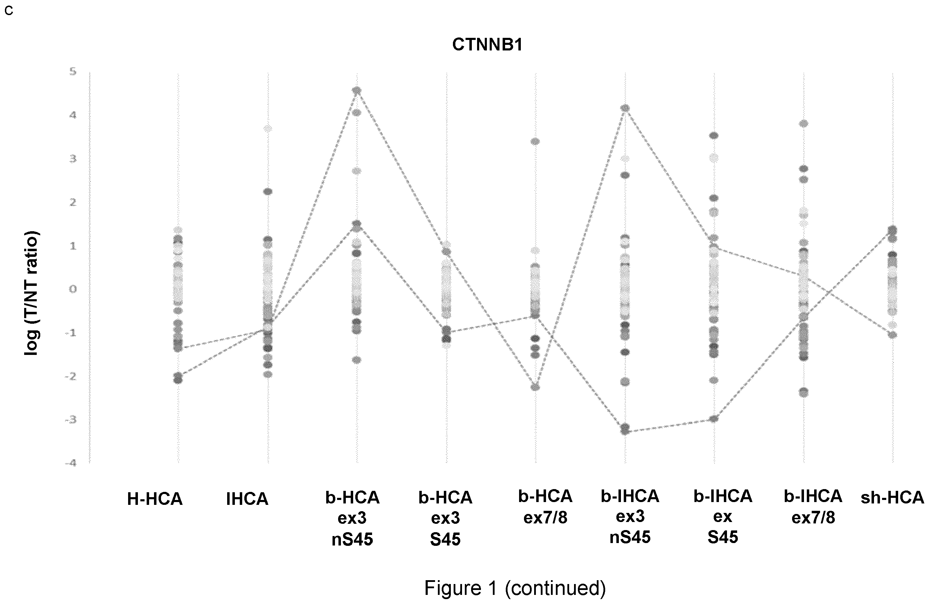

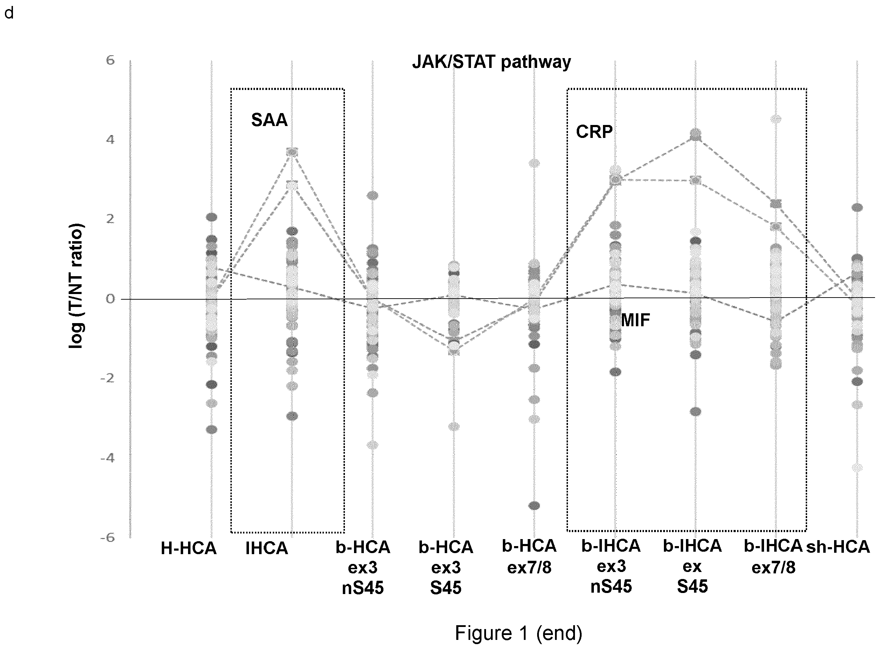

- Figure 1: represents HCA database validation and linking of proteomic profiles with mutations defining HCA subtypes. a: Deregulation of HCA biomarkers (L-FABP, GS, CRP/SAA and ASS1) for each subtype (H-HCA, IHCA, b-HCA ex3 nS45, b-HCA ex3 S45, b-HCA ex7/8, b-IHCA ex3 nS45, b-IHCA ex3 S45, b-IHCA ex7/8, sh-HCA and FNH) in the 60 cases constituting the proteomic database. Each point represents the Tumoral vs Non-Tumoral (T/NT) ratio of a case. The median of T/NT ratios is indicated for each subtype. The line indicates identical expression level between the T and NT liver (T/NT=1). b to d: Deregulation of the proteins frequently functionally associated with the mutated gene in each HCA subtype (H-HCA, IHCA, b-HCA ex3 nS45, b-HCA ex3 S45, b-HCA ex7/8, b-IHCA ex3 nS45, b-IHCA ex3 S45, b-IHCA ex7/8, sh-HCA) for *HNF1A, CTNNB1,* and genes of the JAK/STAT signaling pathway. Immunomarkers of each subtype are highlighted. Examples of proteins with expression linked to mutations in *HNF1A* or *CTNNB1* (ALB and OAT respectively) or proinflammatory cytokines linked to the JAK/STAT signaling pathway (MIF) are highlighted. Gene set enrichment analysis (GSEA) performed from protein expression data identified by proteomics for the pathways and dysfunctions listed by Nault et al ("Molecular Classification of Hepatocellular Adenoma Associates With Risk Factors, Bleeding, and Malignant Transformation". Gastroenterology. 152, 880-894.e6 (2017) ([12])) used to characterize each HCA molecular subtype (data not shown).

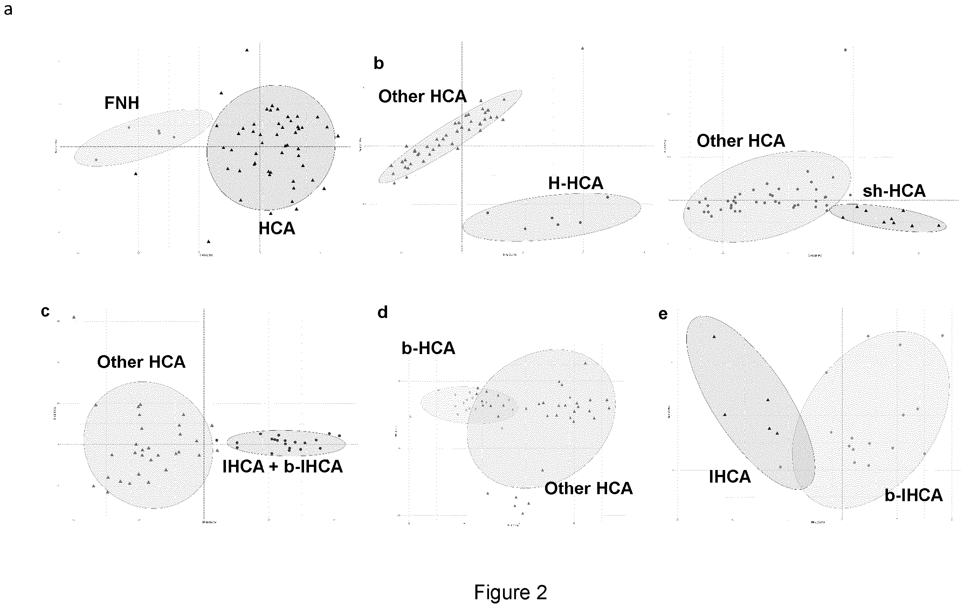

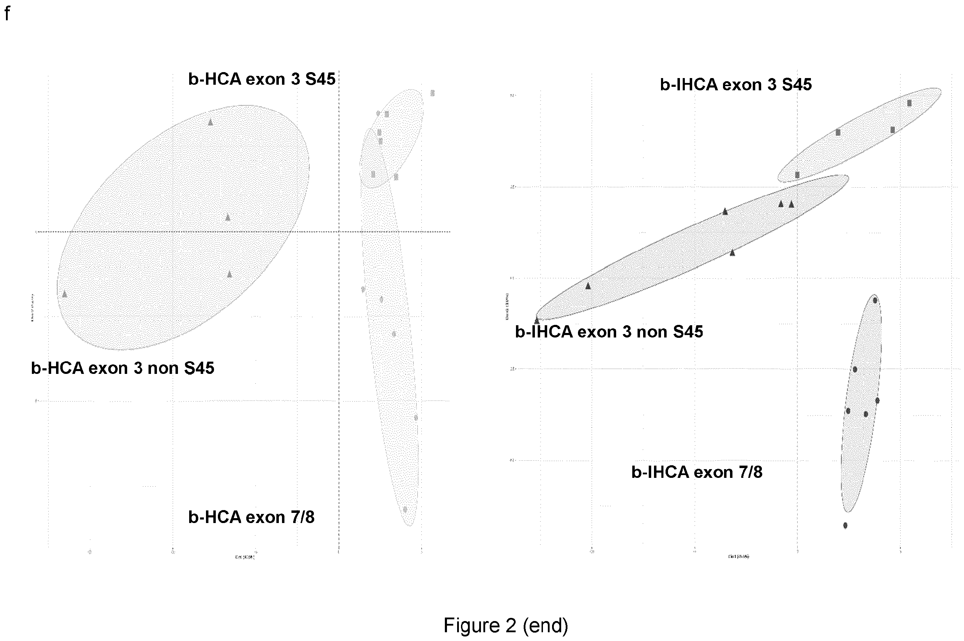

- Figure 2: represents Principal Component Analysis (PCA) comparing the profiles of protein expression deregulation in HCA versus FNH (a) and between the different HCA subtypes (b: H-HCA sh-HCA; c: IHCA+b-IHCA; d: b-HCA; e: IHCA and b-IHCA; f: b-IHCA ex3 S45 and b-HCA ex3 nS45; b-IHCA ex7/8, b-IHCA ex3 nS45 and b-IHCA ex3 S45). Biological pathways associated with HCA subtype-specific profiles. Gene Set Enrichment analyses (GSEA) was carried out on the Ingenuity Pathway Analysis (IPA) database (Canonical Pathways) (data not shown).

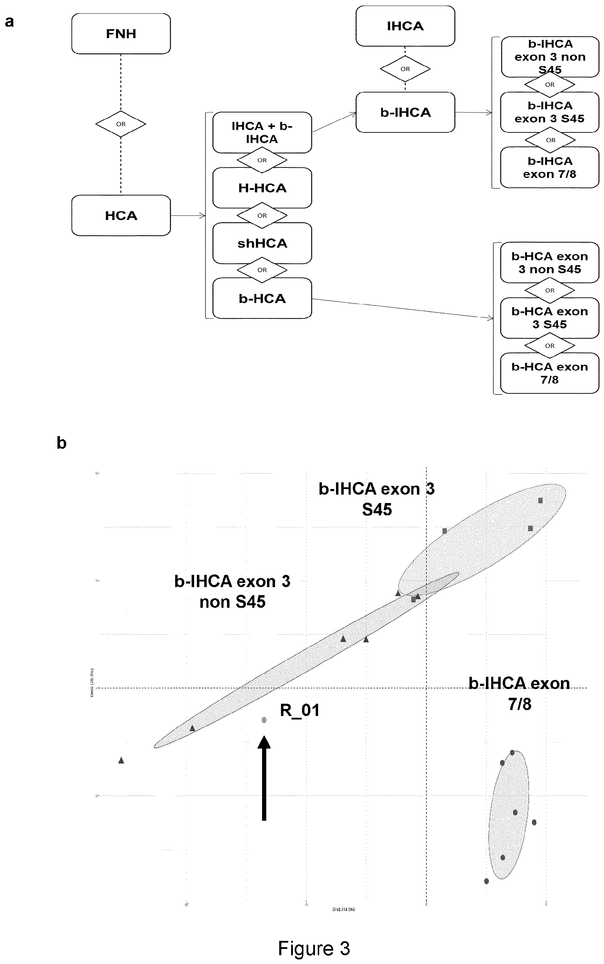

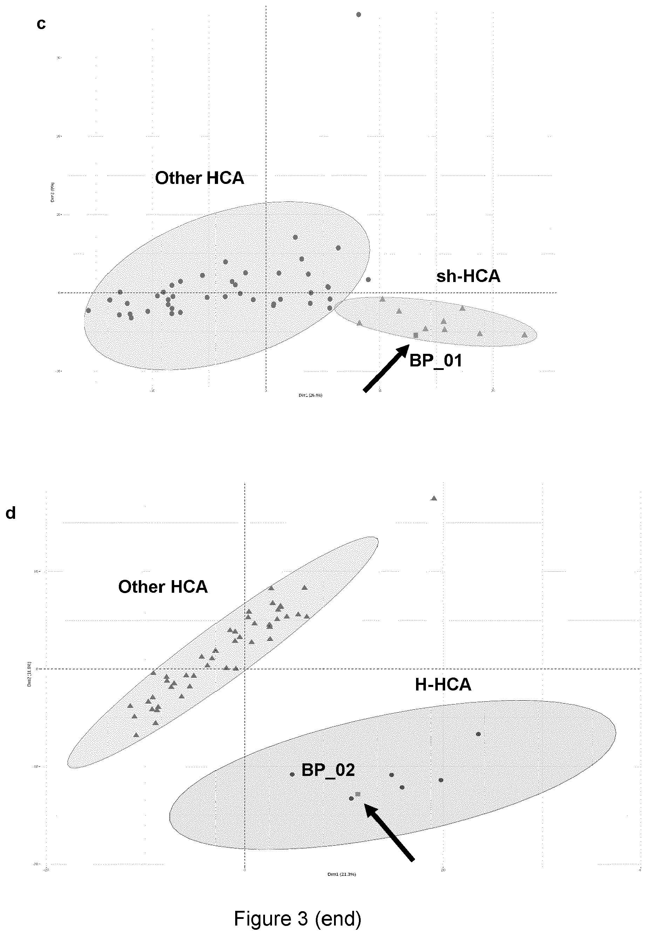

- Figure 3: represents: a Diagnostic algorithm based on sequential proteomic pattern matching. b to d: Example of results that can be obtained using proteomic pattern matching (b (R_01) corresponds to b-IHCA exon 3 non S45, c (BP_01) to sh-HCA, d (BP_02) to H-HCA). The location of each case on the PCA from the reference database is indicated by a red arrow.

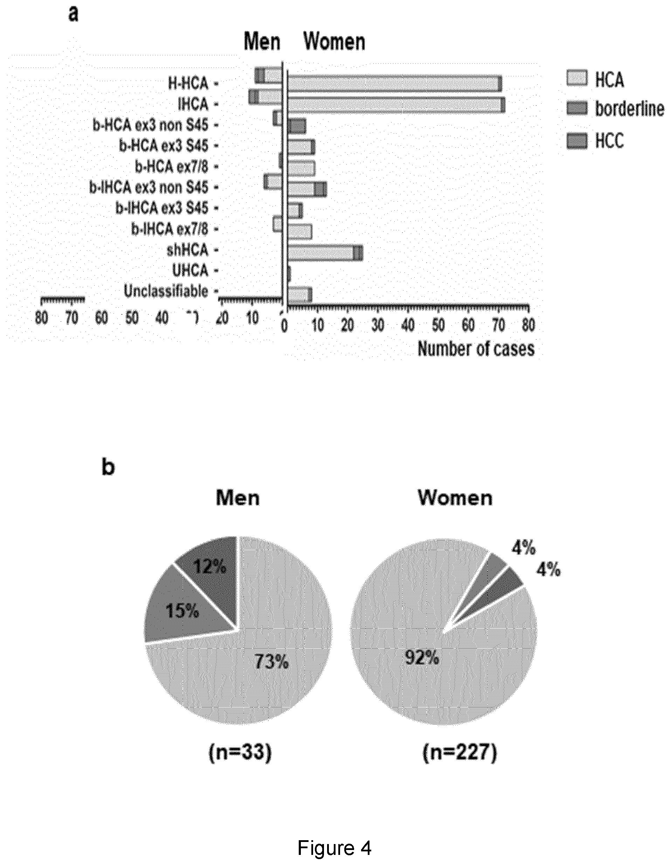

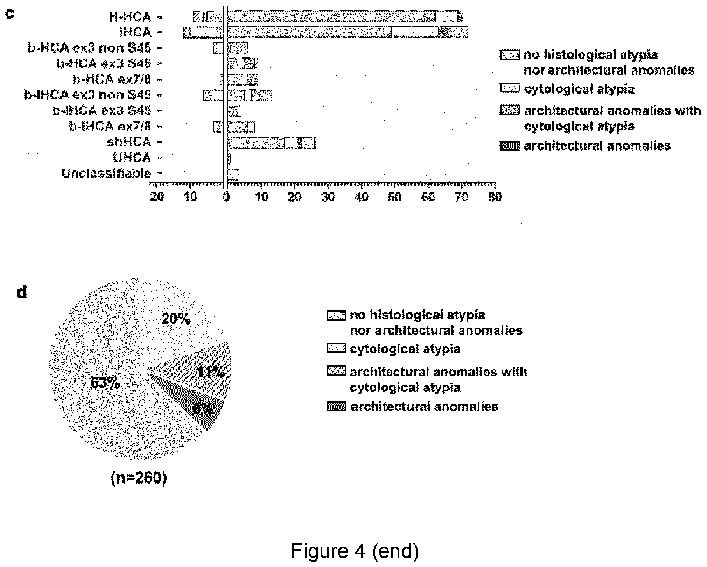

- Figure 4: represents a: Identification of transformed HCA cases classified by molecular subtype. b: Percentage of HCC or "borderline" developed on HCA diagnosed in men versus women in our collection of 260 cases at Bordeaux. c and d: Percentage of HCA cases in our Bordeaux collection with cytological atypia and/or architectural anomalies extracted from pathological reports (d) according to molecular subtype (c).

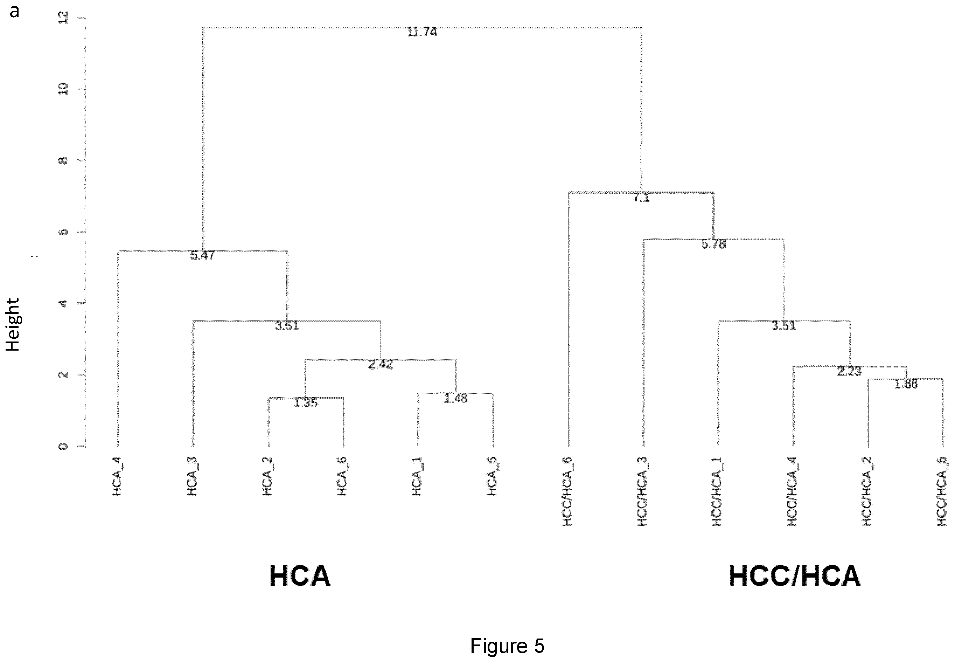

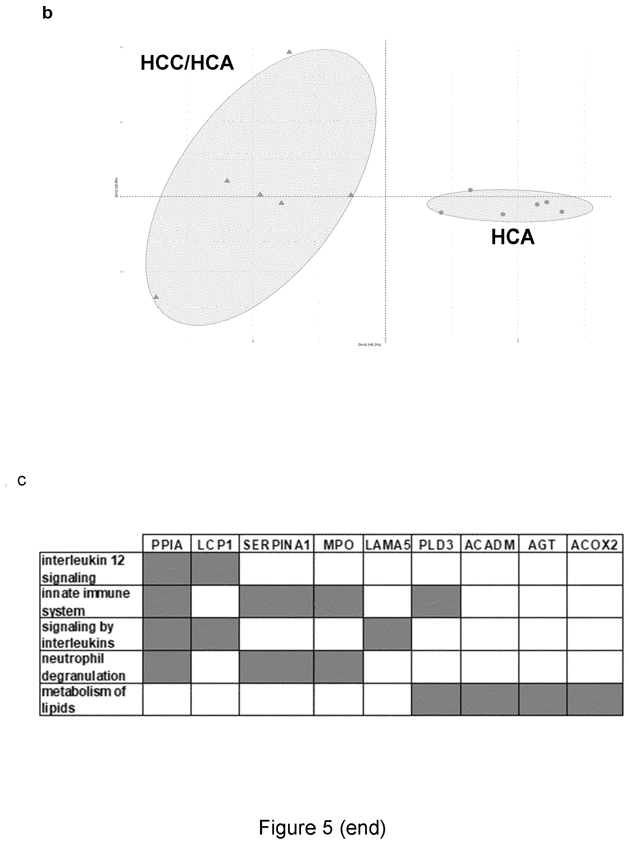

- Figure 5: represents Hierarchical clustering (a) and Principal Component Analysis (PCA) comparing the profiles of protein expression deregulation for HCC developed on HCA (HCC/HCA) and the corresponding HCA (b). Ten proteins were identified and constitute a signature for HCA malignancy. Their levels of deregulation (T/NT ratios) are represented in the form of a heatmap (data not shown) The functional annotations extracted from the Reactome database are listed in table c.

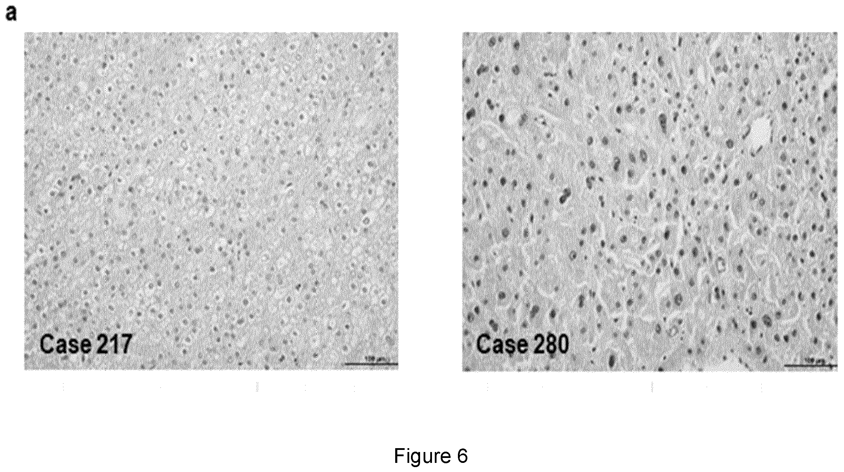

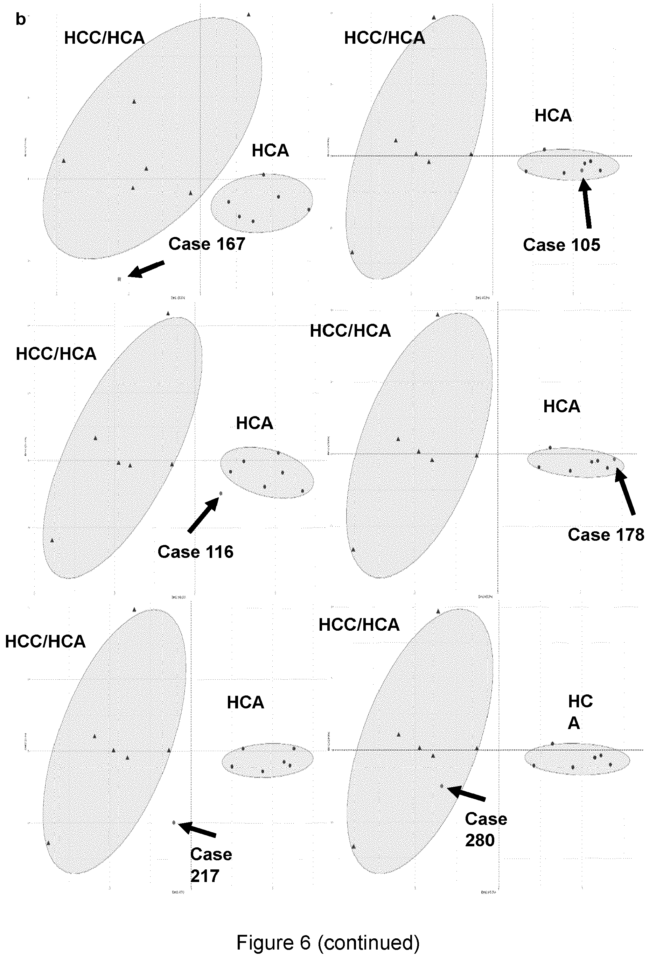

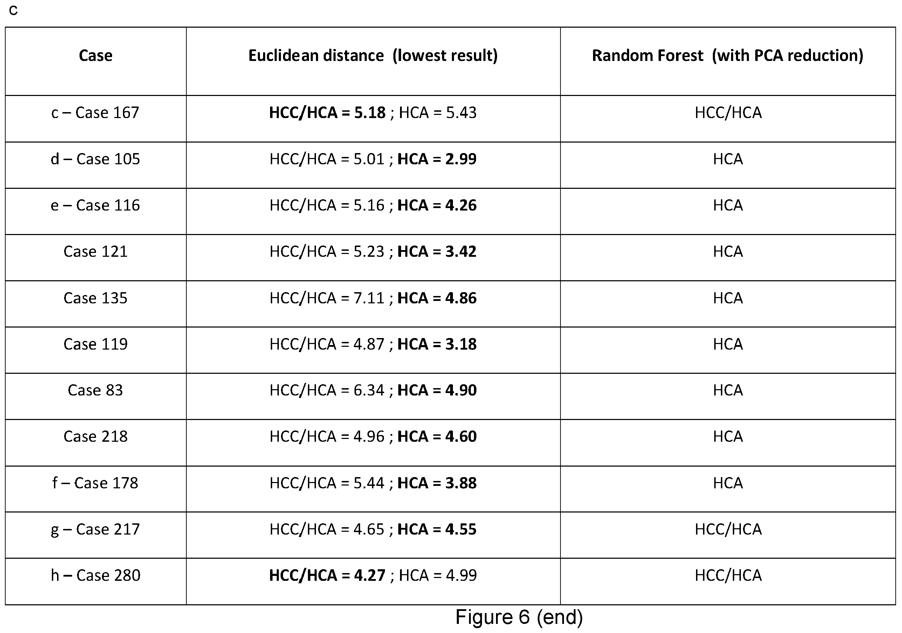

- Figure 6 represents malignancy signature validation on 12 cases. a-b Histological hematoxylin eosin (H&E) staining of 2 cases presenting suspected malignancy : case 217 (left) mild cytological atypia associated with foci of decreased reticulin network (not shown here) leading to the diagnosis of "borderline HCA" and case 280 (right) suspected to be a well differentiated HCC with obvious cytological and architectural atypia; these 2 cases are located on principal component analysis (PCA). (b) Example locations on PCA for validation cases. The location of the tested cases on the PCA is indicated by a green arrow.

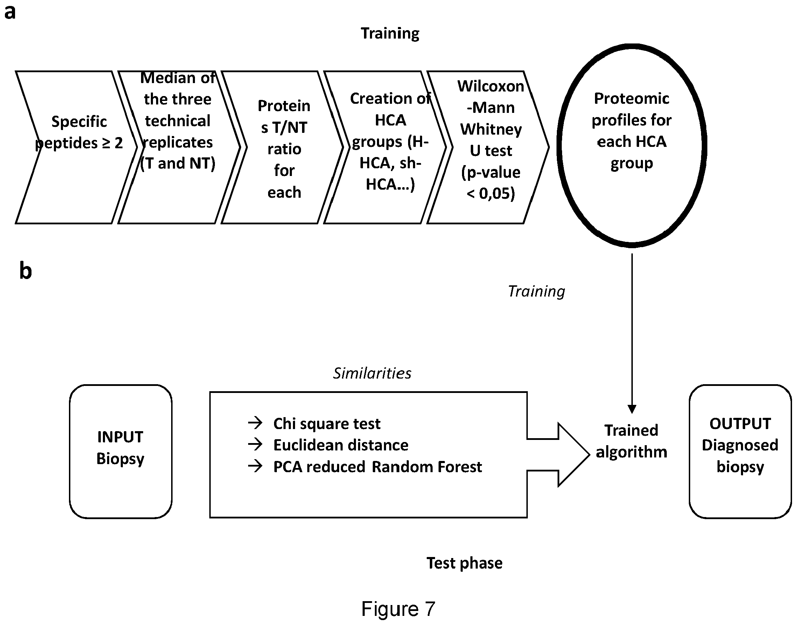

- Figure 7 represents a summary of the phases carried out in the methods of the invention.

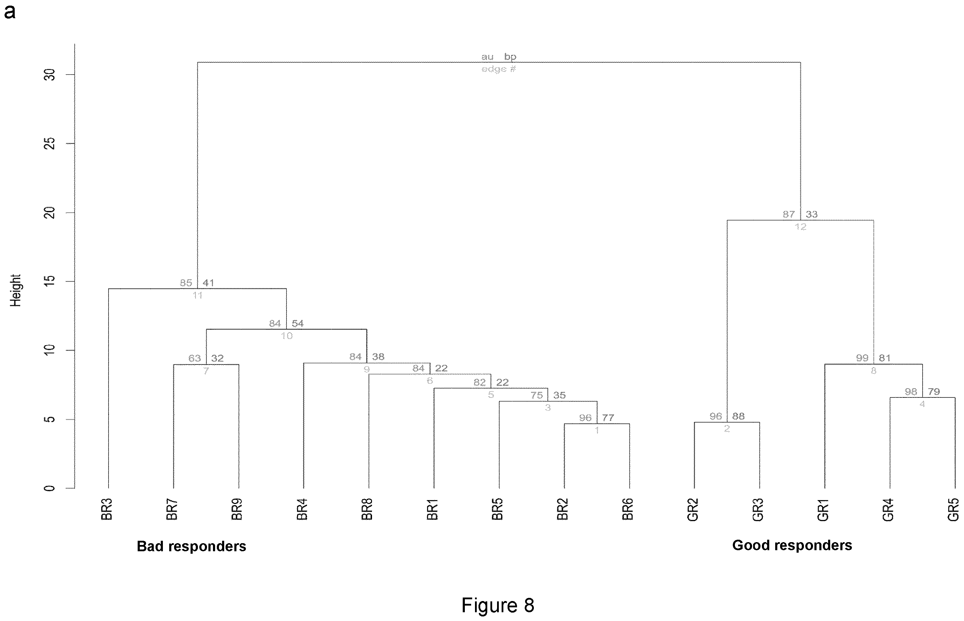

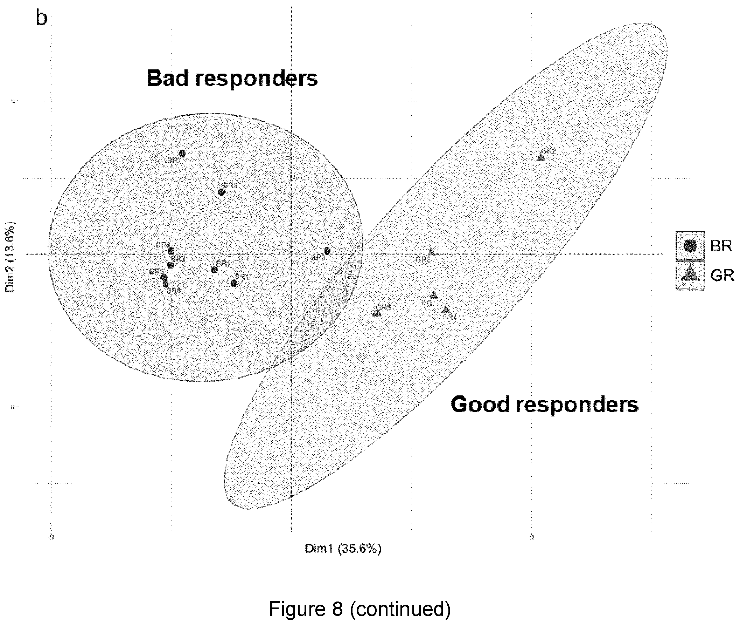

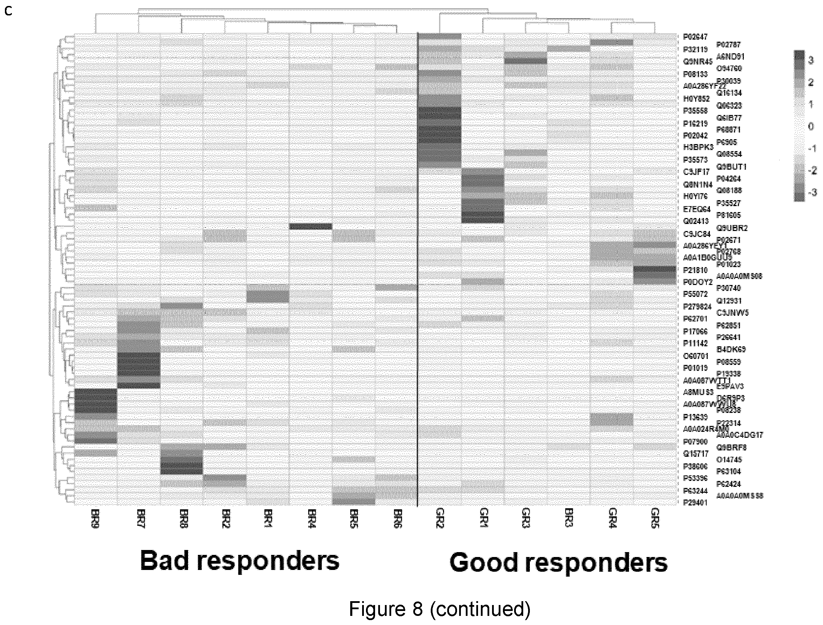

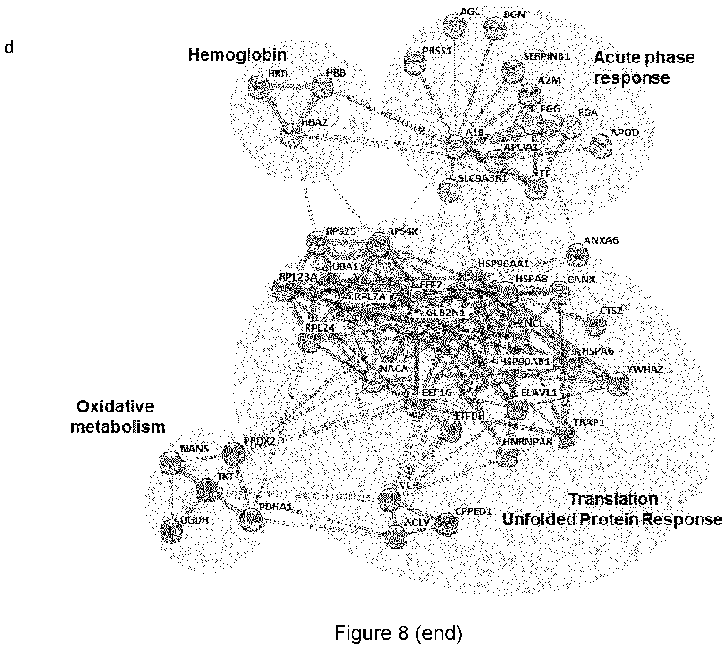

- Figure 8 represents the identification of a proteomic signature predictive of the response of advanced HCC patients to sorafenib treatment. a : Hierarchical clustering of the proteomic profiles of Tumoral vs Non-tumoral (T/NT) expression deregulation in good (GR n=5) or poor responders (BR n=9) to sorafenib. The dataset was reduced to a signature of 77 proteins with significantly different expression between the two groups. Values at the left represent approximately unbiased p-values (AU) and values at the right correspond to boot-strap probability (BP). b : Principal Component Analysis of the proteomic profiles of Tumoral vs Non-tumoral (T/NT) expression deregulation in good (GR n=5) or poor responders (BR n=9) to sorafenib. The dataset was reduced to a signature of 77 proteins with significantly different expression between the two groups. GR patients are represented with orange triangles and BR with blue points. c: Heatmap of the 77 proteins that are significantly differentially expressed between GR and BR for each patient. d : Representation of functional interactions among the 77 proteins of the theranostic signature (made from the String database https://string-db.org/).

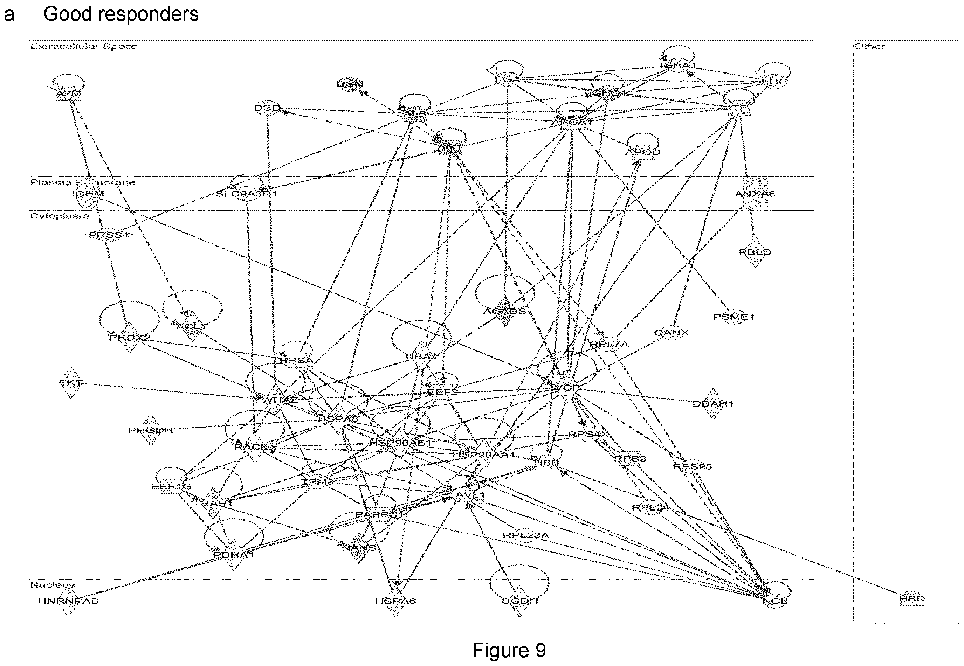

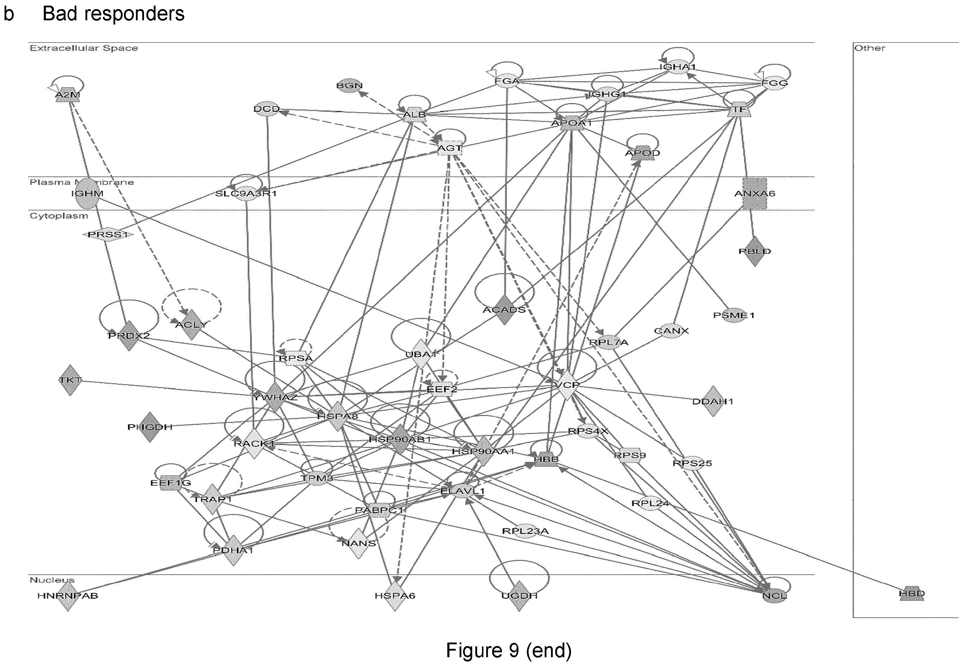

- Figure 9 represents the functional interactions among the 77 proteins of the theranostic signature (made from the Ingenuity Pathway (IPA) database). Colors correspond to the difference in protein expression between tumoral vs. non-tumoral tissue (red=upregulated and green=downregulated) for good responders (a) and poor responders (b).

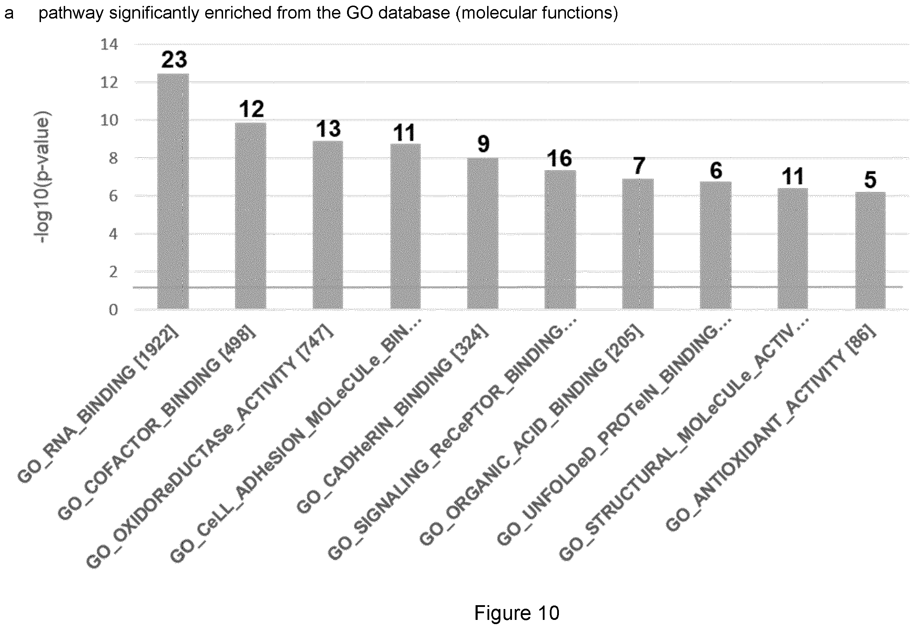

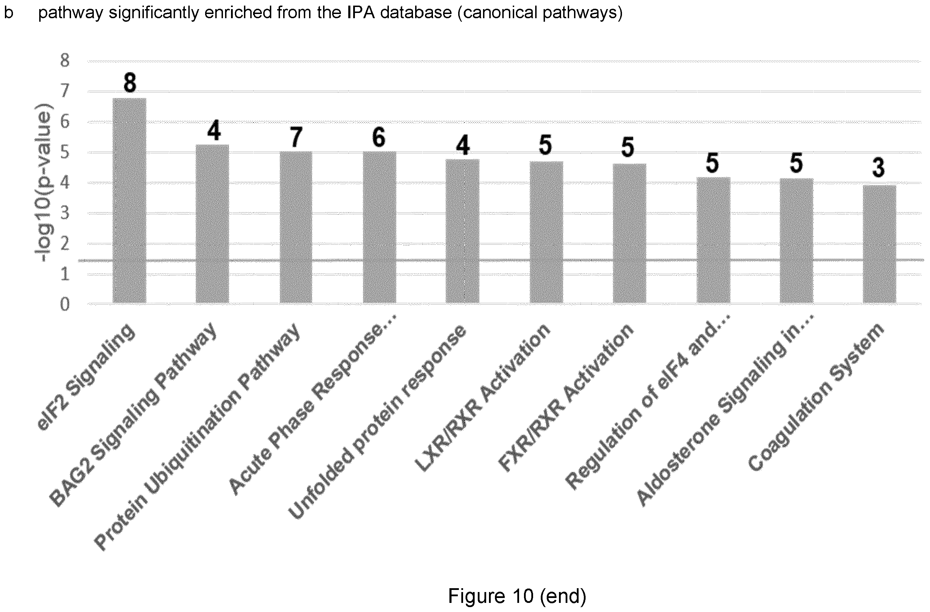

- Figure 10 represents gene set enrichment analysis. The results obtained from the Gene Ontology (molecular functions). a : and Ingenuity Pathways (IPA) (canonical pathways). b : databases using the 77 proteins that were significantly differentially expressed between GR and BR. The y axis represents the negative log10*p*value. The orange line represents the minimum level of significance (p<0.05). The number at the top of each bar corresponds to the number of proteins used to calculate the enrichment for each biological function.

### Examples

### Example 1: Diagnosis of hepatocellular adenoma and malignancy determination by proteomic pattern matching

HCA are rare liver tumors that have been subjected to an extensive and precise genetic, immunopathological, and clinical characterization in the litterature (Zucman-Rossi J. et al.: "Genetic Landscape and Biomarkers of Hepatocellular Carcinoma". Gastroenterology. 149, 1226-1239.e4 (2015) ([13]); Nault et al. ([12])). HCA mainly affects women in their middle ages and men to a smaller extent. The main risk factor for HCA is hormonal exposure to estrogens or androgens, but metabolic, vascular, glycogen storage diseases, and some other rare genetic diseases have also been associated with the development of HCA. Bleeding and malignant transformation into hepatocellular carcinoma (HCC) are the two major complications, both strongly related to the size of the adenoma and its pathomolecular subtype. A resection is usually recommended when the HCA reaches 5 cm and when the β-Catenin pathway is activated. In order to specify the cases with significant clinical risk, HCA have been classified into four groups based on the identification of mutations and on corresponding clinical, histological, and immunohistological characteristic (Nault et al. ([12])) :
(1) H-HCA with inactivating mutations of HNF1A. L-FABP, whose expression is controlled by HNF1A, is not immunodetected in H-HCA tumor cells;
(2) Inflammatory HCAs (IHCA) exhibit various mutations (IL6ST, FRK, STAT3, JAK1, GNAS) or chromosome alterations that activate the JAK/STAT signaling pathway (Nault et al ([8])). Neoplastic hepatocytes show strong and diffuse immunoreactivity for the acute phase inflammatory proteins SAA and CRP;
(3) b-HCA with activating mutations of the CTNNB1 gene encoding β-catenin (exon 3 at various hot spots, including S45 or exon 7/8). The GLUL gene, a β-catenin target, encoding Glutamine Synthetase (GS) is strongly detected in non-S45 exon 3 b-HCA tumors. BHCA exon 3 non-S45 tumors are at high risk of malignant transformation into HCC, particularly if they are associated with mutations in the Telomerase Reverse Transcriptase (TERT) promoter. In addition, half of β-catenin-mutated HCAs also exhibit inflammatory features (b-IHCA);
(4) The last group corresponds to sh-HCA, defined by microdeletions fusing the INHBE promotor with the GLI gene. Sh-HCA is identified by the overexpression of ASS1, and has a high hemorrhage risk which is a major clinical issue (Nault et al. ([12])).

The genotype/phenotype classification of HCA is currently considered as almost complete from a scientific point of view. However, the mechanisms underlying malignant transformation and bleeding remain unknown. In addition, the daily management of patients with HCA remains a challenge for many surgeons and hepatologists. The first challenge is the differentiation of HCA from other benign liver "tumors". These other tumors are characterized as hyperplastic in nature: Focal Nodular Hyperplasia (FNH). For some cases, differential diagnosis between HCA and FNH remains challenging by imaging and biopsy analysis, even for liver pathologist experts. This is notably because GS is also upregulated in FNH. As FNH does not hemorrhage or undergo malignant transformation, resection is not indicated and monitoring is not mandatory. After HCA diagnosis, the second challenge lies with the determination of the risk of transformation into HCC and hemorrhage in order to propose resection when appropriate (transplantation in rare cases), taking into account the risk-benefit ratio associated with surgery.

The aim of this study was to establish, using HCA as a model, the proof of concept that proteomic-based machine learning analysis is operational in routine clinical practice and could provide progression in patient diagnosis. Finally, this type of approach is a breakthrough that is transposable to other pathologies.

### Material and Methods

### Patients

All patients gave informed consent and this study was approved by our local committee "Direction de la Recherche Clinique et de l'Innovation" of Bordeaux University Hospital, Bordeaux Liver Biobank BB-0033-00036.

### HCA database construction

In our cohort of 260 resected HCA, 248 cases corresponded to H-HCA (77), IHCA (82), b-HCA (28), b-IHCA (35), sh-HCA (25) HCA; only 1 case was an unclassified HCA. In 12 cases, subtyping couldn't be assessed for technical reasons. We selected 52 samples that were surgical resections of HCA and representative of all the different subtypes as defined by the 2019 WHO classification20 (6 H-HCA, 5 IHCA, 16 b-HCA (4 b-HCA exon 3 non S45, 5 b-HCA exon 3 S45, 5 b-HCA exon 7/8 plus two cases of b-HCA (1 exon 3 S45 and 1 exon 7/8), sent for second opinion, were added in this collection), 16 b-IHCA (6 b-IHCA exon 3 non S45, 4 b-IHCA exon 3 S45, 6 b-IHCA exon 7/8), 9 sh-HCA). The diagnosis of HCA and HCA subtype was considered as characteristic by the pathologist according to morphology and immunostainings, and results were always confirmed by genotyping.

### Proteomic analysis

Preparation of histological sections, laser microdissection cutting, protein extraction, formalin fixation reversal, preparation of samples for mass spectrometry analysis, mass spectrometry analysis, processing of raw mass spectrometry data, and label-free quantification were carried out as previously described (Sala M, et al. ([1]); Coscia F, et al. ([3])). The mass spectrometry proteomics data have been deposited to the ProteomeXchange Consortium via the PRIDE partner repository.

### Mass spectrometry data processing

The quantitative data were analyzed using the free R software. Proteins identified by mass spectrometry with less than 2 specific peptides were excluded. For each patient, three technical tumour (T) and non-tumour (NT) replicates (from serial sections) were performed. A T/NT ratio from technical replicate medians was calculated for each protein. The Wilcoxon-Mann-Whitney U-test was performed using the R package in order to specifically identify the proteins with significantly different expression (p<0.05) in each group, corresponding thus to the specific proteomic profiles.

### Distance calculations and pattern matching

Analyzes were performed using the free R software. Hierarchical clusterings were represented via the R package and its "hclust" function. The "ward.D" method was used as the agglomeration method and the Euclidean distance was used for the distance calculation. For Principal Component Analysis (PCA), missing values were imputed using the k-Nearest Neighbor (KNN) imputation method from the VIM package. PCA data was calculated and formatted by the PCA and fviz_pca_ind functions in the factoextra and FactoMineR packages. Euclidean distances were calculated using the dist function and Chi-square test of independence using the chisq.test function (both within the R package). The Random Forests were generated using the randomForest function in the random Forest package. The optimal parameters were calculated by the train function in the caret package.

### Integrative biological analyses

Pathway analyses were performed using the Ingenuity Pathway Analysis (IPA) (Qiagen) database. The ten most significantly enriched canonical pathways were selected from each group (HCA subtypes and FNH) and compared. The functional annotations were extracted from the Gene Ontology (biological functions) and Reactome databases using the GSEA (Gene Set Enrichment Analysis) web tool (https://www.gsea-msigdb.org/gsea/index.jsp).

### Selection of cases for validation of proteomic profiles

To validate the diagnostic applicability of the proteomic profiles we identified, we selected a panel of 11 cases of biopsies and 1 resection for sample classification and 4 resections for malignancy status.

### Graphical representations

The graphical representations were formatted using the ggplot2 package and all its dependencies.

### Results

### State-of-art HCA diagnosis in our center

First of all, in order to evaluate HCA diagnosis and to improve it, we reviewed HCA management in our tertiary center (Bordeaux, France). All HCA are routinely classified by histopathological analysis and IHC. Out of a total of 260 resected cases (227 female/33 male) included for study between 1984 and 2020, 71% (185/260 cases) of cases were also analyzed by molecular biology primarily on resected specimen to identify genetic mutations.

Clinical data and patient management history were collected for all cases. Twenty-seven percent (70/260 cases) of cases had also been biopsied prior to surgery. We extracted the medical history of all these cases. Among the 70 biopsies, only 27 (38.6%) were contributory to the diagnosis of HCA and HCA subtyping; these were both determined later on the surgical resection. When biopsy interpretation was not formal, it was either due to technical noncontributory factors, such as lack of interpretable material (8 cases), or to the impossibility of making a standard differential diagnosis with well-differentiated HCC (15 cases) or FNH (3 cases) using standard pathological tools. These data, based on our experience in our tertiary center at Bordeaux, revealed that even for a well-characterized tumor such as HCA and expert pathologists in the field, an additional diagnostic support tool could have been useful for 60% of these diagnostic biopsies.

### Creation of a reference HCA proteomic database

In order to construct a robust database from our collection, we selected 52 HCA surgical resection cases that were representative of all HCA subtypes and FNH. The diagnosis of these resections was considered typical by the pathologist and confirmed by genetic analysis.

Applying our optimized method for proteomic analysis, we compared the relative protein abundances between tumoral (T) and non-tumoral (NT) liver tissues from each patient. We identified and quantified an average of 1604 proteins for each patient. To begin, in order to validate the robustness of our database we quantified the HCA biomarker T/NT ratios used in routine clinical diagnosis. We confirmed the specific and expected deregulation of GS, CRP/SAA, and ASS1 markers in the corresponding neoplasms: b-HCA/b-IHCA exon 3 non S45, IHCA/b-IHCA, and sh-HCA (Figure 1a, b and c), respectively. Loss of L-FABP was only associated with H-HCA (median T/NT=0.04), but we also noticed a decrease in b-IHCA exon 3 non S45 (median T/NT=0.49), b-IHCA exon 7/8 (median T/NT=0.59), and sh-HCA (median T/NT=0.50) (Figure 1a).

We first assumed that the protein signature for each HCA subtype would reflect the underlying mutation(s) that defined them. Therefore, we examined the proteins functionally associated to the underlying mutated genes defining each HCA subtype (HNF1A, CTNNB1, JAK/STAT signaling pathway). Surprisingly, with exception of the validated target proteins currently used as immunomarkers (L-FABP, GS, CRP/SAA), we did not observe major deregulation of the other proteins frequently found associated with each mutation. We illustrate this with the examples of ALB30, OAT31, and MIF32 (Figure 1b, c and d). In addition, we explored the previously described typical/specific pathways for each HCA group from a transcriptomic approach13 (estrogen-associated oral contraceptive pathway predominantly found in IHCA and sh-HCA, tumor steatosis in H-HCA, cholestasis in b-HCA and b-IHCA, development of HCC in b-HCA). We found all these pathways significantly deregulated in all HCA subtypes by means of unsupervised analysis (data not shown). We noted within these results that proteins associated with HCC development were significantly deregulated in all HCA subtypes, suggesting thus that all subtypes can potentially be transformed. At this stage, we concluded that even if a mutation and associated markers can identify a subtype of HCA, this cannot alone define the phenotype.

We therefore investigated the specificities of each HCA subtype in regard to deregulated protein expression. We selected both the significant variations in protein expression between HCA and FNH, and also the significant variations for each HCA group (Figure 2). We analyzed the distances between the HCA groups from each specific protein expression profile. The proteomic profiles of HCA and FNH were quite distinct (Figure 2a). Once we could differentiate HCA and FNH proteomic profiles, we then tested whether it was possible to differentiate the different HCA subtype proteomic profiles. We first separated the most dissimilar profiles: H-HCA and sh-HCA (Figure 2b). Interestingly, the inflammatory protein expression profile was a strong signature shared by inflammatory HCA (IHCA and b IHCA), two groups of which were distinct from the other HCAs (Figure 2c). The proteomic profile of b-HCA was not well distinguished from the other HCAs by PCA (Figure 2d), even following hierarchical clustering of b-HCA. Yet, when IHCA and b-IHCA were specifically compared, their proteomic profiles were markedly different (Figure 2e). Moreover, their protein expression profiles were clearly grouped according to the type of CTNNB1 mutation (exon 3 non S45, exon 3 S45, or exon 7/8) for both b-HCA and b-IHCA (Figure 2f).

These results demonstrated the excellent correlation between the proteomic profiles and the genotype/phenotype classification and revealed the existence of both dominant (sh-HCA, inflammatory H-HCA) and less obvious profiles (CTNNB1 mutations).

We then examined the biological functions that were associated with each specific profile. Gene Set Enrichment Analysis (GSEA) revealed a major difference between FNH and HCA. This was the presence of mitochondrial disorders in HCA, including disruptions in amino acid metabolism that were not significant in FNH (Data not shown). The analysis of the profile of proteomic deregulations associated with each HCA subtype revealed less precise specificities than the genetic classification. On the contrary, we found that most of the proteomic deregulations were shared by the different HCA subtypes (Data not shown). As expected, the most significantly deregulated pathway for H-HCA was the fatty acid betaoxidation pathway related to steatosis, which is characteristically observed in these tumors but was also found in inflammatory HCA. These data may be consistent with the fact that 23/80 cases of IHCA were steatotic in our collection (11 cases presenting steatosis in more than two-thirds of the liver and 12 cases between one- and two-thirds). H-HCAs also showed strong deregulations in amino acid metabolism and xenobiotic metabolism, a significant pathway deregulation shared with sh-HCA, for which we also found the previously described urea cycle deregulation Henriet E, et al. ([4])).

The most significantly enriched pathways for inflammatory HCA (IHCA and b-IHCA) were as anticipated related to activation of the inflammatory response (FXR/RXR activation, acute phase response signaling). Stress-related signals regulating mRNA translation associated with Eif2 signaling pathways were also significantly enriched in inflammatory HCA, similarly to the sh-HCA-specific proteomic profile (Data not shown). In contrast, and unlike sh-HCA, the HHCA-specific proteomic profile exhibited significant protein expression deregulation associated with the estrogen signaling pathway; a feature shared with inflammatory HCAs and to a lower extent with b-HCAs (Data not shown). The b-HCA subtype could not be distinguished by the enrichment of a specific pathway, including even the β-catenin pathway (Data not shown). This integrative analysis revealed that genetic mutations did not translate into a strictly clear functional classification at the protein level.

### A machine learning tool for HCA and FNH diagnosis

Given the statistical analysis of our proteomic database enabled us to distinguish each different HCA subtype, we decided to set up a diagnostic tool. The first important step for clinical application was to define whether the sample corresponded to a HCA or an FNH, and not another type of well-differentiated liver tumor. To examine this, using our proteomic profile identification method we analyzed two other types of well-differentiated liver tumors not included in our database and therefore for which we did not have reference proteomic profiles: a low-grade dysplastic nodule in a cirrhotic liver and a well-differentiated HCC. For both cases, the similarity scores with HCA or FNH were very low, ruling out the possibility of erroneous attribution and indicating that tumors were neither HCA nor FNH. Next, we built an algorithm that integrated first the main HCA groups identified (sh-HCA, HHCA, Inflammatory (IHCA and b-IHCA) and then the secondary proteomic profiles (b-HCA according to the different CTNNB1 mutations) (Figure 3a). The principle being thus to sequentially compare the HCA subtype reference proteomic profiles with new cases in order to diagnose their HCA subtype (Figure 3b, c, and d). We used three approaches to calculate the similarities: (1) a Chi-square test to compare upregulated proteins (T/NT ratio≥1.5), down-regulated proteins (T/NT ratio≤0.67), and non-regulated proteins (T/NT ratio ≥0.67 and ≤1.5) between the different reference proteomic profiles; (2) a calculation of Euclidean distance between each group from the PCA; (3) a Random Forest analysis on PCA-reduced datasets. We applied them to a panel of 11 biopsies and 1 resection of FNH and HCA (any subtype) to test the advantages and limitations of each analysis.

Three out of the 12 cases were typical b-HCA exon 3 non S45, sh-HCA and H-HCA (R_01, BP_01, and BP_02, respectively) and did not present any difficulty in classification (Figure 3b, c, d and e). The other nine cases consisted of a representative panel of biopsies diagnosed on morphology and IHC in our tertiary center. The mass spectrometry quantification of single HCA biomarkers was noncontributory to the subtyping of any of these cases. However, the proteomic profiles identified by the three different mathematical approaches were consistent with the clinicopathological interpretation for three cases (BP_03, _04 and, _05). For two other cases, (BP_06 and _07), the Random Forest and Euclidean distance (BP_06) or the Chi-square test (BP_07) matched with the pathological diagnosis; however, one test gave a different result for each case. For these two cases the Random Forest gave the correct diagnosis but these cases were less typical and did not perfectly match with the HCA in the database. Another case (BP_08) was a b-HCA exon 7/8 subtype subsequently identified by genetic analysis on resection. Random Forest and Euclidean distance firmly recognized it as a b-HCA exon 7/8 subtype, but Chi-square testing grouped it with the very similar b-HCA exon 3 S45 group. Finally, three biopsies without subsequent resection (BP_09, _10, and _11) and for which the pathologist could not differentiate IHCA from b-IHCA (exon 7/8 or exon 3 S45) were found to group with b-IHCA exon 7/8 or exon 3 S45 profiles.

In conclusion, these results demonstrate that proteomic pattern matching can differentiate HCA from FNH and assign the molecular HCA subtype. In case of difficulties with respect to atypical cases, proteomic profiling can bring additional clues that support diagnosis.

### Identification of the proteomic profile of malignancy for transformed HCA

Despite a well-established pathomolecular classification, HCA management remains delicate for many surgeons. In 1996, Ault G.T., et al. ("Selective management of hepatic adenomas". Am. Surg. 62, 825-829 (1996) ([14])) had already proposed the resection of symptomatic HCA or HCAs larger than 5 cm, but despite the advent of molecular and immuno-pathological classification, nothing has substantially changed since these clinical recommendations. Indeed, it is still currently impossible to limit surgical resection exclusively to HCA with malignant potential since it is in fact very difficult to identify. To confirm this, we noticed that the emergence of molecular classification has not modified HCA management according to subgroup type in the clinical practice of our tertiary center. This is despite a slight tendency to perform more preoperative biopsies.

Hence, improving classification is not sufficient for changing the management of patients with HCA. However, malignant transformation is a major complication of HCA and we wanted to determine if our proteomic profile-based tool could help to address this issue. Analysis of the clinical data from our collection of cases determined that the majority of HCA subgroups could undergo malignant transformation (Figure 4a), and even if b-HCA exon 3 non S45 subtype showed the highest risk of malignant transformation, a non-negligible proportion of cases from other subgroups could be concerned (up to 13.6% for sh-HCA) (Figure 4a). It is noteworthy that the interpretation of histological features of malignancy can be challenging, depends on the expertise of the pathologist, and can be observer-dependent. In half of these cases we noticed a reservation on the pathologist's behalf. This was described by the term "borderline" and defined as a neoplasm that was no longer characteristic of HCA, while at the same time presenting atypical foci lacking some characteristics of definite HCC (Figure 4a and b). In addition to the clinical and imaging context, the assertion of HCC diagnosis in histology is based on a set of features that include morphological criteria, such as cytonuclear atypia (Figure 4c and d), pseudoglandular formation, trabecular thickening, stroma invasion, reticulin network disorganization/reduction, but also immunohistochemical criteria (including immunomarker positivity, for example Glypican 3 (GPC3), Heat Shock Protein 70 (HSP70), and molecular features including TERT promotor mutation). These criteria are not completely specific and sensitive, but rather quite subjective. Furthermore, these criteria could be different in cases of HCC developed on HCA given they were primarily defined in de novo HCC or in HCC arising in dysplastic nodules in cirrhotic livers (Figure 4c). This is the reason why discriminating the difference between a very well-differentiated HCC and a HCA versus the diagnosis of HCA-related malignant foci can be challenging, especially for some well differentiated β-Catenin activated tumors. The term HUMP (Hepatocellular Neoplasm of Uncertain Malignant potential) has been proposed to group these. Therefore, it appeared pragmatic to identify a proteomic signature of malignancy in order to improve patient management.

### Identification of the HCC developed on HCA malignancy profile

To identify proteins related to malignancy in HCA, we selected six cases of surgical resection that presented definite HCC developed on HCA (we refer to this as "HCC/HCA") from the different HCA subtypes (2 b-HCA exon 3 non S45, 1 b-IHCA exon 3 S45, 1 UHCA, 2 sh-HCA). As previously, we compared the T/NT ratios of six HCA and their corresponding HCC/HCA and isolated a significant proteomic profile consisting of ten proteins. These proteins allowed the perfect separation of malignant and benign tissues, both by hierarchical clustering (Figure 5a) and PCA (Figure 5b). Moreover, the protein expression deregulations within each group were homogeneous (Data not shown). Note that biomarkers routinely used to identify malignancy (GPC3, HSP70) in other clinicopathological contexts were not part of this signature. GPC3 was not identified by proteomic analysis in any cases of HCC developed on HCA. HSP70, identified by proteomic analysis in all cases, was not upregulated in any cases of HCC developed on HCA.

Half of the extracted functional annotations of the malignancy protein profiles were associated with immune response and immune-related cell activation (MPO, PRDX2, LCP1, PPIA, SERPINA1, PLD3), Interleukin-12 signaling (PPIA, LCP1), and neutrophil degranulation (PPIA, SERPINA1, MPO) (Figure 5c). Among these ten proteins, none were part of the known β-catenin functional environment.

We then wanted to validate this proteomic profile of HCA transformation with a set of several new cases. The Chi-square scoring method was not applicable for a profile consisting of only ten proteins. Even though the differences between Euclidean distances were small, they appeared applicable and so we used distance calculation in addition to Random Forest after reduction by PCA. For validation we first tested our ten-protein proteomic profile of transformation on one positive control of HCC developed on HCA and 7 cases of benign HCA from our database.

### Validation with a control HCC developed on HCA

Case 167 was an epileptic male with HCC developed on HCA without any histological ambiguity. The HCC presented a well-differentiated hepatocellular proliferation with some nuclear atypia and bile pigments in the tumor cells, and an irregular and reduced reticulin network. IHC showed strong and diffuse GS staining, diffuse CD34 staining in sinusoids, 5-10% of cells were MIB1-positive in some areas, and GPC3 staining was foci-specific. Molecular analysis identified a mutation in exon 3 (hot spot T41) of the CTNNB1 gene. The proteomic profile of this case was closer to the confidence ellipse of the transformed cases, confirmed by the Random Forest test and the smaller Euclidean distance for the HCC group (5.18 for HCA vs 5.43 for HCC) (Figure 6b and c). Our proteomic profile thus allowed confirmation of the malignancy of this case of transformed HCA.

### Validation with benign controls

Next, we selected seven cases of HCA that were not questionable regarding their diagnosis of benign tumors by histology (Figure 6b, c). The proteomic profiles of all these tumors were associated with benign HCA, both by PCA confirmed by the Random Forest test and the smallest Euclidean distance for the HCA group (Figure 6b and c). The proteomic profile allowed confirmation of the benign nature of all cases of HCA lacking histological features of malignant transformation.

### Cases with doubt on the diagnosis of malignancy

We then selected cases with a management history raising doubts concerning the diagnosis of malignancy. The first case was suspected at the time of biopsy analysis and corresponds to an incidentally discovered HCA that developed on a fibrotic non-alcoholic steatohepatitis (NASH). The interpretation of the preoperative biopsy by the pathologist was not capable of clearly distinguishing an HCA from a very well-differentiated grade 1 HCC. The conclusive histological analysis of the surgical resection was clear on the benign nature of this HCA, which was later identified as a sh-HCA. In order to reproduce a clinical context, we analyzed the proteome of the preoperative biopsy that had initially raised the doubt. Unambiguously, the proteomic profile was closer to the confidence ellipse of HCA. This was confirmed by Random Forest testing and the smallest Euclidean distance for HCA (2.99 for HCA vs 5.01 for HCC) (Figure 6b and c).

Next, we selected a "borderline" case that manifested intratumoral bleeding by imaging in a growing and unclassified HCA. In 2016, the state of knowledge in histology did not allow the classification of this highly vascularized HCA, and neither for steatotic nor inflammatory HCA. The presence of an intratumoral hematoma made interpretation difficult and it was challenging to distinguish between HCA and HCC due to the presence of cytological atypias and abnormal sinusoids (CD34-positive and reduced reticulin staining). IHC did not indicate a known subtype (decreased but not loss of L-FABP (which is usually the case in sh-HCA), GS-negative, and weakly CRP-positive). Molecular biology analysis found a posteriori diagnosis was a sh-HCA. The proteomic profile was very clearly associated with the HCC group, as confirmed by the Random Forest test. However, the Euclidean distance calculation did not allow a decision between the two groups (4.55 for HCA vs 4.65 for HCC). For this specific case, proteomics provided additional quantitative features that allowed the positioning of this neoplasm on a progressive scale between HCA and HCC (Figure 6a, b and c).

The last case (Figure 6b and c) was a female initially managed remotely for a benign 10 cm liver tumor. Histological analysis of the resection specimen was a challenge for the pathologists: unclassifiable HCA with negativity of all routinely used IHC markers, combined with diffuse cytological atypia and an abnormal reticulin network. This thus raised the diagnosis of probable HCC, but without HCC-favoring IHC markers (GPC3- and MIB1-negative) and a negative molecular analysis (TERTnegative). The proteomic profile clearly allowed the classification of this case with the HCC developed on HCA group (4.27 for HCA vs 4.99 for HCC).

In view of these results on this validation patient set, the proteomic malignant profile was effective for the determination of HCA engaged in a process of transformation.

Within this study we offer a complete tool for HCA diagnosis that can determine HCA subtype and a score reflecting its level of malignant transformation.

### Conclusion

Proteomic analysis is the next "omic" step following on from genomics and transcriptomics to improve patient management. Until now, proteomic profiling of FFPE tissue had never been used in clinical practice, especially in the tumor pathology field.

We have carried out pioneering work using proteomic pattern matching for diagnosis from FFPE tissues. The main principle of our tool is based on the comparison of a proteomic profile extracted from a biopsy or surgical specimen with a reference database composed of proteomic profiles from completely characterized patients. We have developed and adapted the analytical process for operational routine analysis. We use a small amount of tissue corresponding to 1 mm² on 3x5 µm section cuts, thus not depleting the sample and it can be reused. A complete analysis can be achieved within a week and so it remains compatible with the deadlines imposed by clinical practice. Moreover, the proteomic data generated remains a resource that can be re-exploited for further applications (prognosis, theranostics).

The main advantage of working on a profile of protein deregulation (tumoral vs non-tumoral) is the ability to directly access the tumor identity by reducing inter-individual variation, making the analysis efficient despite small patient collections. This also allows the association of profiles with functional biological pathways, the control of biological relevance, and identification of new targets. The additional advantage of working on a profile of protein expression deregulation versus spectral intensity profiles is that the construction of the reference database is not dependent on mass spectrometer type. Indeed, our method can be implemented on sites with different equipment, contrary to radiomics that encounter major obstacles concerning the heterogeneity between devices used for image acquisition.

The molecular classification of HCA has been subject to many updates over the last ten years and is now considered almost established Nault J-C, et al ([12]). It must be noticed that while the enrichment of the molecular classification has led to a better understanding of HCA physiopathology, it has not modified the management of patients. We identified two main issues: (1) classification based on biopsies and (2) identification of the malignancy status. Indeed, for several reasons the diagnosis on biopsies using classical approaches is not evident. Moreover, the fact that potentially all HCA can transform can induce doubt at the time of diagnosis, especially for a pathologist who is not specialized in benign liver tumors. A suspicion of HCC drastically alters the medical and surgical treatment and follow-up. Indeed, the surgical strategy is not thought-out in the same way; a simple enucleation of the tumor is sufficient when dealing with HCA. On the contrary, the surgical management of HCC consists, at best, in an anatomical resection (removing the corresponding portal branch), and when this it is not possible, the surgeon must strive to have an optimal margin of 2 cm.

For all these reasons, we first generated an HCA database for HCA subtype proteomic classification. Secondly, we identified a protein profile that directly diagnoses malignant transformation regardless of HCA subtype. This profile was correlated with immune activation in response to cell degeneration, making sense for tumors transforming or differentiated tumors with active anti-tumor immunity. It is likely that the β-catenin pathway did not emerge in our malignancy signature given it was identified across all HCA subtypes. This validated signature allows us to define the HCA transformation status. Machine learning is an emerging and promising disciplinary field for clinical data interpretation. In our study, we tested different statistical analyses for calculations of similarity (difference score calculation: Chi-square test on simplified data, Euclidean distance calculation after PCA, Random Forest on data reduced by PCA). Without preconceived ideas, we wanted to use some of the analyses to provide evidence on the similarities of the proteomic profile we were testing with respect to the reference groups. Although Random Forest was proven the most reliable, these methods were all successful. To interpret this, if the three mathematical approaches lead to the same conclusion, it is evidence of robustness. On the other hand, if they conflict with respect to atypical cases, it is necessary to be more cautious and consider the analyzes as additional features in diagnosis. Ongoing case addition to our HCA database will make the diagnosis more and more precise.

Thereafter, our methodology could be implemented to answer other critical clinical questions in the liver field, such as well-differentiated HCC for which differential diagnosis can be confusing in a common clinical context. This could define whether the malignancy signature we identified is strictly related to HCA or is common to other more frequent etiologies and backgrounds.

From a broader point of view, the clinical recommendations for performing liver biopsies on liver tumors and peritumoral tissues are not systematic and must follow a logical benefit over risk assessment. Indeed, this invasive procedure is not insignificant and can lead to complications, sometimes serious, such as hemorrhage and tumor cell spread. The value of biopsy performance is also under debate given advances in imaging. Indeed, radiologists are able to make increasingly accurate diagnoses, even by elimination. This already aids in management and especially in the decision for resection. For these reasons, the role of biopsy for patients with liver disease is one of the most important considerations among hepatologists. Nonetheless, information provided by the proteomic profile could reverse the benefit-risk ratio of performing liver biopsy, making a real difference in clinical practice and pave a way for personalized medicine. For example, malignancy diagnosis could indeed be very useful for dysplastic liver nodules found in cirrhotic livers because the malignancy conditions patient eligibility for transplantation. In the future, proteomic profiles could also be used to identify elements of response or non-response to anti-cancer treatments, for example in patients with advanced HCC for which the therapeutic arsenal has recently been expanded. Outside the liver, proteomic profiling can improve the performance of biopsies in the oncology field: lung cancer, low-grade gliomas, sentinel lymph nodes in breast cancer for which very little biopsy material is available and IHC is difficult to interpret.

In conclusion, we show that proteomic profiling could give biopsy a more important status in the patient management process. We show proof of concept with the diagnosis and subtyping of HCA and the evaluation of its malignant transformation, which is available for a transfer to routine clinical care. This implies the integration of proteomic analysis into the clinics and the addition of proteomic results into patient pathology reports as part of the evidence-based care bundle. Proteomic analysis will bring pathology services into a new analytical era.

### Example 2: Theranostic approach in advanced HCC using biopsies proteome profiling: sorafenib as proof of concept

Liver cancer is a major health problem and is the second leading cause of cancer death worldwide, with more than 850,000 new cases each year. In western countries, there has been an increase in the number of cases over the past 20 years. Among primary liver cancers, hepatocellular carcinoma (HCC) is the most common (Ferlay J, et al. "Cancer incidence and mortality worldwide: sources, methods and major patterns in GLOBOCAN". Int. J. Cancer. 136:E359-386 (2012) ([7])), that usually develops on cirrhosis, and exceptionally in healthy liver. While cirrhosis surveillance programs can detect a large number of early-stage HCC, for which curative treatment is available (surgical resection, percutaneous destruction, liver transplantation), 50 to 70% of cancers are diagnosed late. Known for its extremely poor prognosis, advanced HCC can only be treated with regional (arterial chemoembolization) or systemic palliative therapies.

The role of liver biopsy in the diagnosis and management of HCC has always long been questioned. The risk of complications, such as tumor seeding and bleeding, as well as availability of performant non-invasive diagnosis by imaging, have so far limited the use of liver biopsies in the management of HCC. However, it is now widely accepted that these risks are uncommon, manageable, do not affect the course of the disease and should therefore not be considered as a reason to avoid a biopsy. On the other hand, the risk of insufficient sampling is also highlighted by many studies. This is probably due to the lack of precise technology and also because DNA and RNA analyses are more efficient on frozen samples than on fixed biopsies that correspond to the material in routine clinical use. However, these tumor samples are undoubtedly underrated and underused as fixed biopsies contain valuable information that could reverse the current risk/benefit ratio (Di Tommaso L, et al. "Role of liver biopsy in hepatocellular carcinoma". World. J. Gastroenterol. 25:6041-6052 (2019) ([8]).

In most countries, sorafenib is approved as the standard first-line therapy for advanced HCC because of its demonstrated survival benefits despite a very low number of responders and significant side effects (Hollebecque A, et al. "Systemic treatment of advanced hepatocellular carcinoma: from disillusions to new horizons". Eur. J. Cancer. 51:327-339 (2015) ([9]). Since the approval of sorafenib in advanced HCC, several phase III clinical trials failed to demonstrate a superior survival benefit (Hollebecque et al. ([9])). Furthermore, a phase III study has shown the non-inferiority of lenvatinib compared to sorafenib in overall survival with equivalent safety and tolerability profiles justifying its use today in the first line treatment (Kudo M, et al. "Lenvatinib versus sorafenib in first-line treatment of patients with unresectable hepatocellular carcinoma: a randomised phase 3 non-inferiority trial". Lancet. 391:1163-1173 (2018) ([10]). According to the clinical recommendations, patients for whom sorafenib or lenvatinib have been ineffective or not tolerated, can benefit from second-line options that have recently emerged (including cabozantinib, ramucirumab, nivolumab, pembrolizumab. The arrival on the market of these new drugs with proven efficacy has been a real revolution in HCC palliative care.

In addition, after initial promising results of immunotherapy treatments (nivolumab, pembrolizumab), the combination of atezoliumab (anti-programmed death-ligand 1 (PDL1)) and bevacizumab (anti-vascular endothelial growth factor (VEGF)) has recently shown a dramatic benefit in first line compared to standard of care (sorafenib) and will be a new standard in first line in the near future. This strengthened therapeutic arsenal is a considerable advance that already makes it possible to increase median overall survival from 6 months to up to more than 26 months in patients with advanced HCC and preserve liver function. However, the therapeutic strategy corresponding to the choices of the first-line treatment and/or therapeutic sequences are not well-defined.

Sorafenib is a multi-targeted kinase inhibitor (Raf kinase, VEGF-R1 and R3, c-kit and RET) that directly targets both tumor cells (cell proliferation inhibition) and endothelial cells (angiogenesis inhibition) (Ferlay et al. ([7])). For more than 10 years, studies have multiplied to identify genetic profiles, clinical parameters, prognostic or predictive blood or tissue biomarkers of response to sorafenib in HCC (Zucman-Rossi et al. [(13])) (Marisi G, et al. "Ten years of sorafenib in hepatocellular carcinoma: Are there any predictive and/or prognostic markers?". World J. Gastroenterol. 24:4152-4163 (2018) ([15]); Pinyol R, et al. "Molecular predictors of prevention of recurrence in HCC with sorafenib as adjuvant treatment and prognostic factors in the phase 3 STORM trial". Gut. 68:1065-1075 (2019) ([16])). Some results have been encouraging (higher pre-treatment phospho-ERK levels associated with a longer delay progression time (Abou-Alfa GK, et al. "Phase II study of sorafenib in patients with advanced hepatocellular carcinoma". J. Clin. Oncol. 24:4293-4300 (2006) ([17])), high expression of c-Met predicting therapeutic efficacy (Chu JS, et al. "Expression and prognostic value of VEGFR-2, PDGFR-β, and c-Met in advanced hepatocellular carcinoma". J. Exp. Clin. Cancer Res. 32:16 (2013) ([18])), higher expression of phospho-c-Jun in patients who do not respond to treatment (Hagiwara S, et al. "Activation of JNK and high expression level of CD133 predict a poor response to sorafenib in hepatocellular carcinoma". Br. J. Cancer. 106:1997-2003 (2012) ([19])); however, none have been validated to date.

We previously combined laser capture and mass spectrometry analysis to thoroughly analyze tumor proteomic profiles (Henriet E, et al. ([4]); Vial G, et al. "Antigenic Mimicry in Paraneoplastic Immune Thrombocytopenia". Front Immunol. 10:523 (2019) ([20])). This technological process, compatible with the analysis of formalin-fixed and paraffin-embedded tissues (FFPET) and with very small quantities of material such as diagnostic biopsies, has already enabled us to identify a biomarker now used for the diagnosis of sonic hedgehog hepatocellular adenomas associated with bleeding risk (Henriet E, et al. ([4]); Nault J-C, et al. ([12]); Sala M, et al. ([1])).

In this study, using diagnostic biopsies, this *in situ* proteomic strategy was applied to develop a theranostic strategy. The aim was to identify markers predicting response of advanced HCC to treatments using analysis of HCC proteomic profiling on fixed biopsies before sorafenib treatment and comparison of protein expression deregulations between tumor and adjacent tissues of good and poor responders to sorafenib. This process and proof of concept, carried out with sorafenib, is applicable to other HCC treatments, and is also transposable to other pathologies.

### Material and Methods

### Patients

Access to the biological resources needed to carry out this study was guaranteed by the presence of the Magellan Digestive Oncology Department (Pessac, France) and in partnership with the Centre de Ressources Biologiques of Bordeaux (request n°160, assignment n°676 made to the CRB on 2017/06/09).

Patients with advanced HCC treated with sorafenib were selected and divided into two groups according to their response to treatment (see Table 1 below).

**Table 1: Clinical characteristics of patients selected for proteomic analysis**

| Demographic and clinical characteristics | Good responders | Poor responders |
|---|---|---|
| | (No = 5) | (No = 9) |
| Baseline Characteristic | | |
| Age at introduction of Sorafenib, years, mean | 65 | 68 |
| Male, No. | 5 | 8 |
| Female, No. | | 1 |
| Positive for viral hepatitis, No. | 1 | 4 |
| Hepatitis B | 0 | 1 |
| Hepatiitis C | 1 | 3 |
| Alcohol use, No. | 4 | 2 |
| NAFLD, NASH, No. | 1 | 1 |
| No chronic liver disease, No | 1 | 3 |

| Pre-sorafenib BCLC stage | | |
|---|---|---|
| BCLC stage B, No | 1 | 2 |
| BCLC stage C, No | 4 | 7 |

| Pre-sorafenib therapies | | |
|---|---|---|
| TACE, No. | 1 | 1 |
| Surgery, No | 1 | 3 |
| Radioembolisation, No. | 0 | 1 |

| Pre-sorafenib liver characteristics | | |
|---|---|---|
| Fibrosis (F1-F4), No. | 4 | 6 |
| F1-F2 | - | 3 |
| F3-F4 | 4 | 3 |
| Steatosis, No. | - | 2 |
| Normal liver, No. | 1 | 3 |

| Pre-sorafenib HCC characteristics | | |
|---|---|---|
| Differenciation | | |
| Moderatly - Well, No. | 2 | 7 |
| Little, No | 2 | 2 |
| Unknown | 1 | - |
| Macroscopic vascular invasion, No. | 2 | 4 |
| Metastatic spread, No. | 1 | - |

| AFP at diagnosis | | |
|---|---|---|
| Median, ng/mL | 7000 | 13674 |

The response was based on radiological response criteria and/or alpha-fetoprotein (AFP) levels assessed in first two months after treatment initiation. A good response corresponded to a radiological tumor regression by >30% and/or decrease in AFP by >50%. A radiological tumor progression >20% corresponded to a poor response.

Each patient must have had a biopsy including sufficient tumor and non-tumor tissue (>1mm2) for subsequent proteomic analysis by mass spectrometry, prior to the start of treatment.

### Laser capture

The slides from each patient were reanalyzed and the hematoxylin eosin saffron staining was used for histopathological examination to determine the areas of interest to be dissected. Tumoral (T) and non-tumoral (NT) liver of the same surface area and homogeneous constitution (absence of fibrosis) were then selected under the supervision of anatomopathologists (BLB; PBS).

One square millimeter of tumoral and non-tumoral tissue previously chosen on H&E stained slides were micro-dissected from a FFPE 5 µm thickness section with a PALM type 4 (Zeiss) laser micro-dissector. The same area was cut into serial sections and treated in the same way to form 3 technical replicates.

### Sample preparation for proteomic analysis

Fixation reversion and protein extraction from tissues sections, in gel proteic digestion, peptide extraction from gel and peptide sample preparation for LC-MS/MS analysis were performed as previously described (Henriet E, et al. ([4])).

### Mass spectrometry analysis

Online nano LC-MS/MS analyses were performed using an Ultimate 3000 RSLC Nano-UPHLC system (Thermo Scientific, USA) coupled to a nanospray Q-Exactive hybrid quadrupole-Orbitrap mass spectrometer or a nanospray Orbitrap Fusion^{™} Lumos^{™} Tribrid^{™} Mass Spectrometer (Thermo Scientific, USA) as decribed previously (Henriet E, et al. ([4])).

### Database search, MS results processing and quantification

For protein identification, the Mascot 2.5 algorithm available with Proteome Discoverer 1.4 Software (Thermo Fisher Scientific Inc.) was used. It was used in batch mode by searching against the UniProt Homo sapiens database (71 525 entries, Reference Proteome Set, release 2017_12) from http://www.uniprot.org/ website. Two missed enzyme cleavages were allowed. Mass tolerances in MS and MS/MS were set to 10 ppm and 0.02 Da. Oxidation of methionine, acetylation of lysine and deamidation of asparagine and glutamine were searched for dynamic modifications. Carbamidomethylation on cysteine was searched as static modification. Raw LC-MS/MS data were imported into Proline Studio (Bouyssié D, et al. "Proline: an efficient and user-friendly software suite for large-scale proteomics". Bioinforma. Oxf. Engl. 36, 3148-3155 (2020) ([11])) for feature detection, alignment, and quantification. Protein identification was only accepted with at least 2 specific peptides with a pretty rank=1 and with a protein FDR value less than 1.0% calculated using the "decoy" option in Mascot 1. Label-free quantification of MS1 level by extracted ion chromatograms (XIC) was carried out using the parameters previously described (Henriet E, et al ([4])). Protein abundances were normalized using the "median ratio" option of Proline Studio.

### Bioinformatic analysis

Protein differential expression between tumor and non-tumor samples for each patient was analyzed by computing log-ratios of the median of normalized protein abundances for each pair of tumor / non-tumor samples.

In order to reveal potential biomarkers that distinguish between good responders (GR) and bad responders (BR) to sorafenib, a t-test (Welch's t-test that does not assume equal population variance) was applied. The threshold for the significance of the adjusted probability value (p-value) was set at 0.005, resulting in a predictive signature constituted by 77 proteins.

Using this relevant dataset, principal component analysis (PCA) was performed using FactoMineR and Factoextra available in R. Hierarchical clustering was also performed using the multi-scale bootstrap algorithm available in R package pvclust and the parameters set to the Euclidian distance and Ward's minimum variance clustering method with squared dissimilarities. Pvclust computes p-values for each cluster using bootstrap resampling techniques. It provides two types of p-values which enable cluster confidence to be interpreted: "AU" for Approximately Unbiased p-value and "BP" for Bootstrap Probability. AU is computed using multi-scale bootstrap resampling, while BP is produced using normal bootstrap resampling.

In order to find pathways which were significantly deregulated between GR and BR, a Gene Set Enrichment Analysis on the 77 proteins signature was performed against the Gene Ontology (GO) biological process database and the functional interactions listed in the string (https://string-db.org) and Ingenuity Pathways (Qiagen) databases were analyzed.

### Results

### Clinico-pathological features in patients and analytical strategy

Given the very low proportion of good responders to sorafenib (2% objective response in the SHARP test) and the fact that not all patients are biopsied before treatment, the first challenge of this study was to collect enough responders for whom the tumor and the non-tumor liver tissue could be compared before treatment. 5 good responders (3 cases from Bordeaux University Hospital, 1 case from Bayonne University Hospital and 1 case from Pau University Hospital) were collected and compared to 9 bad responders (from Bordeaux University Hospital). The initiation dates for sorafenib treatment were from August 2012 to December 2017. The analysis was performed on FFPET liver biopsies taken prior to starting sorafenib treatment. In addition to the three technical replicates designed to limit variability, the tumor vs non-tumor protein expression ratio was analyzed for each patient in order to smooth the background noise due to the inter-individual variability and thus acquire more statistical power. This methodology allowed to obtain robust data, even on a small cohort (Henriet E, et al. ([4])). The clinical characteristics of the selected patients are detailed in the Table 1 above. The analysis strategy has been designed to limit the bias due to this unavoidable variability (Table 1).

Tumor proteomes differentiate between good patients and bad responders

An average of 982±329 proteins per case was identified and quantified for a total of 2790 proteins identified (with more than 2 specific peptides). On an average, 12.9% of identified proteins were upregulated (T/NT ratio ≥2) and 19.7% were downregulated (T/NT ratio ≤0.5) for good responders; 18.9% of identified proteins were upregulated and 24.3 % were downregulated for bad responders (see Table 2 below).

**Table 2: Protein identification and quantification of each diagnostic biopsy. Tumoral to Non-tumoral (T/NT), good responders (GR) bad responders (BR)**

| Patients | Number of identified proteins (nb specific peptides≥2) | Number of variants (T/NT Ratio ≥2 or ≤0,5) | Number of proteins upregulated in T (T/NT Ratio ≥2) | Number of proteins downregulated in T (T/NT Ratio ≤0,5) | proteins upregulated in T (%) | proteins downregulated in T (%) |
|---|---|---|---|---|---|---|
| GR_P1 | 642 | 265 | 88 | 177 | 0,14 | 27,57 |
| GR_P2 | 1000 | 273 | 185 | 88 | 18,50 | 8,80 |
| GR_P3 | 914 | 122 | 67 | 55 | 7,33 | 6,02 |
| GR_P4 | 1335 | 662 | 219 | 443 | 16,40 | 33,18 |
| GR_P5 | 1212 | 548 | 270 | 278 | 22,28 | 22,94 |
| **Average GR** | **1020±269** | **374±223** | **166±86** | **208±157** | **12,93±9,02** | **19,70±11,83** |
| BR_P1 | 906 | 327 | 112 | 215 | 12,36 | 23,73 |
| BR_P2 | 976 | 507 | 250 | 257 | 25,61 | 26,33 |
| BR_P3 | 603 | 121 | 65 | 56 | 10,78 | 9,29 |
| BR_P4 | 1417 | 396 | 175 | 221 | 12,35 | 15,60 |
| BR_P5 | 1463 | 728 | 333 | 395 | 22,76 | 27,00 |
| BR_P6 | 1367 | 705 | 283 | 422 | 20,70 | 30,87 |
| BR_P7 | 563 | 254 | 137 | 117 | 24,33 | 20,78 |
| BR_P8 | 808 | 389 | 159 | 230 | 19,68 | 28,47 |
| BR_P9 | 549 | 320 | 117 | 203 | 21,31 | 36,98 |
| **Average BR** | **961±372** | **416±200** | **181±89** | **235±116** | **18,88±5,60** | **24,34±8,26** |

To identify a proteomic signature differentiating good and bad responders, an adjusted Wilcoxon t-test was applied comparing the T/NT ratios of the two groups. The threshold for the significance of the adjusted p-value was set at 0.005, resulting in a 77-protein signature (see Table 3 below).

**Table 3: List of the 77 proteins significantly different in expression between good responders (GR) bad responders (BR) to sorafenib with associated p value (Wilcoxon test).**

| | | | | | |
|---|---|---|---|---|---|
| P01023 | A2M | Alpha-2-macroglobulin | 1,52 | 0,52 | 1,59E-02 |
| P16219 | ACADS | Short-chain specific acyl-CoA dehydrogenase, mitochondrial | 0,33 | 0,16 | 4,80E-02 |
| P53396 | ACLY | ATP-citrate synthase | 0,69 | 2,65 | 3,81 E-02 |
| P35573 | AGL | Glycogen debranching enzyme | 0,78 | 0,18 | 2,83E-02 |
| P01019 | AGT | Angiotensinogen | 0,31 | 1,14 | 4,85E-02 |
| B4DK69 | AKR1C2 | Aldo-keto reductase family 1 member C2 | 0,44 | 1,59 | 4,44E-02 |
| A0A0A0MSS8 | AKR1C3 | Aldo-keto reductase family 1 member C3 | 1,05 | 2,53 | 6,06E-03 |
| P02768 | ALB | Serum albumin | 2,13 | 0,72 | 1,90E-02 |
| P08133 | ANXA6 | Annexin A6 | 1,39 | 0,42 | 4,24E-02 |
| P02647 | APOA1 | Apolipoprotein A-I | 1,36 | 0,53 | 1,01 E-02 |
| C9JF17 | APOD | Apolipoprotein D | 1,25 | 0,40 | 3,03E-02 |
| A6ND91 | ASPDH | Putative L-aspartate dehydrogenase | 1,12 | 0,15 | 1,12E-02 |
| P38606 | ATP6V1A | V-type proton ATPase catalytic subunit A | 0,58 | 1,66 | 1,59E-02 |
| Q9BUT1 | BDH2 | 3-hydroxybutyrate dehydrogenase type 2 | 1,45 | 0,24 | 9,52E-03 |
| P21810 | BGN | Biglycan | 3,92 | 0,51 | 2,38E-02 |
| P27824 | CANX | Calnexin | 0,95 | 1,43 | 1,20E-02 |
| Q9BRF8 | CPPED1 | Serine/threonine-protein phosphatase CPPED1 | 0,78 | 1,50 | 1,67E-02 |
| Q9UBR2 | CTSZ | Cathepsin Z | 0,86 | 0,30 | 4,20E-02 |
| P81605 | DCD | Dermcidin | 1,21 | 0,70 | 4,04E-03 |
| O94760 | DDAH1 | N(G),N(G)-dimethylarginine dimethylaminohydrolase 1 | 1,24 | 0,58 | 1,90E-02 |
| Q08554 | DSC1 | Desmocollin-1 | 2,76 | 0,69 | 1,55E-03 |
| Q02413 | DSG1 | Desmoglein-1 | 1,44 | 0,81 | 1,09E-02 |
| P26641 | EEF1G | Elongation factor 1-gamma | 1,02 | 2,26 | 4,20E-02 |
| P13639 | EEF2 | Elongation factor 2 | 1,06 | 1,34 | 4,20E-02 |
| Q15717 | ELAVL1 | ELAV-like protein 1 | 1,08 | 1,68 | 4,85E-02 |
| Q16134 | ETFDH | Electron transfer flavoprotein-ubiquinone oxidoreductase, mitochondrial | 0,83 | 0,30 | 1,59E-02 |
| P02671 | FGA | Fibrinogen alpha chain | 1,43 | 1,25 | 4,20E-02 |
| C9JC84 | FGG | Fibrinogen gamma chain | 1,43 | 1,13 | 6,99E-03 |
| Q6IB77 | GLYAT | Glycine N-acyltransferase | 0,33 | 0,12 | 4,51 E-02 |
| H3BPK3 | HAGH | Hydroxyacylglutathione hydrolase, mitochondrial | 0,63 | 0,28 | 3,03E-02 |
| P69905 | HBA1 | Hemoglobin subunit alpha | 1,14 | 0,42 | 1,90E-02 |
| P68871 | HBB | Hemoglobin subunit beta | 1,13 | 0,37 | 2,90E-02 |
| P02042 | HBD | Hemoglobin subunit delta | 1,20 | 0,31 | 2,90E-02 |
| D6R9P3 | HNRNPAB | Heterogeneous nuclear ribonucleoprotein A/B | 0,97 | 1,87 | 1,01 E-02 |
| P07900 | HSP90AA1 | Heat shock protein HSP 90-alpha | 1,13 | 2,21 | 1,20E-02 |
| P08238 | HSP90AB1 | Heat shock protein HSP 90-beta | 1,12 | 3,27 | 6,99E-03 |
| P17066 | HSPA6 | Heat shock 70 kDa protein 6 | 0,82 | 1,44 | 3,36E-02 |
| P11142 | HSPA8 | Heat shock cognate 71 kDa protein | 1,15 | 1,81 | 1,90E-02 |
| A0A286YEY1 | IGHA1 | Immunoglobulin heavy constant alpha 1 | 1,29 | 0,79 | 4,20E-02 |
| A0A0A0MS08 | IGHG1 | Immunoglobulin heavy constant gamma 1 | 2,06 | 0,75 | 1,21 E-02 |
| A0A1B0GUU9 | IGHM | Immunoglobulin heavy constant mu | 1,47 | 0,58 | 4,51 E-02 |
| P0DOY2 | IGLC2 | Immunoglobulin lambda constant 2 | 3,07 | 0,72 | 7,94E-03 |
| P04264 | KRT1 | Keratin, type II cytoskeletal 1 | 1,87 | 0,80 | 2,90E-02 |
| H0YI76 | KRT5 | Keratin, type II cytoskeletal 5 | 2,14 | 0,70 | 6,22E-03 |
| Q8N1N4 | KRT78 | Keratin, type II cytoskeletal 78 | 1,45 | 0,82 | 2,90E-02 |
| P35527 | KRT9 | Keratin, type I cytoskeletal 9 | 2,45 | 0,83 | 2,00E-03 |
| E9PAV3 | NACA | Nascent polypeptide-associated complex subunit alpha, muscle-specific form | 0,97 | 2,19 | 4,00E-03 |
| Q9NR45 | NANS | Sialic acid synthase | 2,07 | 0,96 | 2,38E-02 |
| P19338 | NCL | Nucleolin | 1,24 | 3,17 | 4,80E-02 |
| A0A087WTT1 | PABPC1 | Polyadenylate-binding protein | 0,99 | 1,72 | 6,99E-03 |
| P30039 | PBLD | Phenazine biosynthesis-like domain-containing protein | 0,96 | 0,09 | 2,38E-02 |
| P35558 | PCK1 | Phosphoenolpyruvate carboxykinase, cytosolic [GTP] | 0,42 | 0,03 | 3,17E-02 |
| P08559 | PDHA1 | Pyruvate dehydrogenase E1 component subunit alpha, somatic form, mitochondrial | 1,05 | 1,73 | 1,21 E-02 |
| A0A286YF22 | PHGDH | D-3-phosphoglycerate dehydrogenase | 0,73 | 0,19 | 2,90E-02 |
| P32119 | PRDX2 | Peroxiredoxin-2 | 1,17 | 0,38 | 1,90E-02 |
| E7EQ64 | PRSS1 | Trypsin-1 | 1,29 | 0,77 | 3,17E-02 |
| Q06323 | PSME1 | Proteasome activator complex subunit 1 | 1,12 | 0,59 | 6,06E-03 |
| P63244 | RACK1 | Receptor of activated protein C kinase 1 | 0,90 | 1,29 | 4,80E-02 |
| A8MUS3 | RPL23A | 60S ribosomal protein L23a | 0,90 | 1,29 | 4,24E-02 |
| C9JNW5 | RPL24 | 60S ribosomal protein L24 | 0,81 | 1,21 | 6,22E-03 |
| P62424 | RPL7A | 60S ribosomal protein L7a | 0,91 | 1,69 | 3,03E-02 |
| P62851 | RPS25 | 40S ribosomal protein S25 | 0,74 | 1,26 | 1,09E-02 |
| P62701 | RPS4X | 40S ribosomal protein S4, X isoform | 0,85 | 1,30 | 4,00E-03 |
| A0A024R4M0 | RPS9 | 40S ribosomal protein S9 | 0,85 | 1,45 | 4,80E-02 |
| A0A0C4DG17 | RPSA | 40S ribosomal protein SA | 0,93 | 1,19 | 1,20E-02 |
| P30740 | SERPINB1 | Leukocyte elastase inhibitor | 1,05 | 2,01 | 3,03E-02 |
| 014745 | SLC9A3R1 | Na(+)/H(+) exchange regulatory cofactor NHE-RF1 | 0,86 | 1,36 | 3,03E-02 |
| H0Y852 | SUCLG2 | Succinate--CoA ligase [GDP-forming] subunit beta, mitochondrial | 1,42 | 0,68 | 1,21 E-02 |
| P02787 | TF | Serotransferrin | 1,32 | 0,66 | 1,20E-02 |
| Q08188 | TGM3 | Protein-glutamine gamma-glutamyltransferase E | 1,63 | 0,49 | 7,94E-03 |
| P29401 | TKT | Transketolase | 0,91 | 2,82 | 6,06E-03 |
| A0A087WWU8 | TPM3 | Tropomyosin alpha-3 chain | 1,08 | 1,71 | 3,81 E-02 |
| Q12931 | TRAP1 | Heat shock protein 75 kDa, mitochondrial | 0,76 | 1,70 | 8,66E-03 |
| P22314 | UBA1 | Ubiquitin-like modifier-activating enzyme 1 | 0,89 | 1,28 | 2,90E-02 |
| 060701 | UGDH | UDP-glucose 6-dehydrogenase | 0,80 | 2,35 | 4,00E-03 |
| P55072 | VCP | Transitional endoplasmic reticulum ATPase | 0,95 | 1,22 | 4,20E-02 |
| P63104 | YWHAZ | 14-3-3 protein zeta/delta | 1,46 | 2,75 | 1,20E-02 |

The two groups of patients appeared to be well separated both in semi-supervised hierarchical and in principal component analyses of differential protein expression profiles. (Figure 8a, b and c) Three-quarters of the proteins were functionally bound according to string protein and ingenuity pathways databases (Figure 8d and Figure 9).

The biological pathways associated with this theranostic signature were explored by gene set enrichment analyses against the GO-database (molecular functions) and against the ingenuity pathways database (canonical pathways) (Figure 10a). These two statistical analyses were complementary and revealed the enrichment of following pathways: (1) the Unfolded Protein Response (UPR) including the EIF2 pathway (phosphorylation of EIF2 being required in the UPR), the protein ubiquitination pathway and the stress response involving the BAG2 signaling pathway, (2) acute phase signaling, (3) oxidative stress and (4) cell adhesion (Figure 8c).

A higher expression of UPR-related pathways was observed in poor responders as well as proteins associated with oxidative stress, whereas good responders were more likely to express proteins related to the acute inflammatory response phase (Figure 10b).

### Conclusion

Despite many studies, the lack of predictive markers for response to sorafenib in advanced HCC still remains a big challenge. These predictive markers could change the way patients are treated, especially due to availability of new treatments (Marisi G, et al. ([15]); Pinyol R, et al., ([16])). So far, three different therapeutic options are available in first-line for advanced HCC, sorafenib, lenvatinib, and more recently the combination atezoliumab / bevacizumab. It is obvious that patients will respond differently to these treatments. Other points to consider are the adverse effects of this drugs and their significant cost for public health. Consequently, it is necessary to reach out to precision medicine to determine the optimal treatment for each patient. All information necessary for this theranostic strategy are available in fixed diagnostic biopsies.

Using our technological process that combines laser capture and mass spectrometry, we could access protein expression deregulation profile from this routine biological sample available in clinic. Our analysis revealed there was a tendency for overexpression of UPR proteins in poor responders prior to treatment. Several studies have previously shown that sorafenib could activate the PERK and IRE1α branches of the UPR in HCC cells but that intrinsic activation is not an indicator of response of sorafenib in advanced HCC. However, UPR has already been described as a determinant of cancer cell response to various cancer therapies. Our results indicate that intrinsic activation of the UPR, prior to treatment, may contribute to a lack of HCC sensitivity to sorafenib treatment. This needs further analysis and validation but could offer a new pharmacological option.

Among the pathways that could be theranostic indicators, we also identified proteins linked to oxidative stress. The upregulation of these proteins could reduce sensitivity to sorafenib treatment in poor responders. Our results obtained in patients corroborate a previous *in vitro* study that showed that sorafenib enhanced reactive oxygen species (ROS) production by HCC cells. These innate deregulations would confer an immediate advantage to the tumor, opposing resistance mechanisms that can be acquired during treatment.

We also found proteins associated with cell adhesion, including BGN among the most discriminating proteins. Indeed, BGN expression is higher in good responders. It is known that sorafenib induces, *in vitro,* a significant reduction in the expression of genes associated with, among other things, cell adhesion (Cervello M, et al. "Molecular mechanisms of sorafenib action in liver cancer cells". Cell Cycle. 11:2843-2855 (2012) ([25])). Moreover, a tumor proteomic analysis comparing protein expression before and after treatment in a non-responder cohort revealed significant enrichment of adhesion pathways. Proteins such as vimentin and extracellular matrix proteins such as fibronectin and vitronectin were upregulated. In addition, adhesion junction molecules were rather subject to negative regulation during treatment (Dazert E, et al. "Quantitative proteomics and phosphoproteomics on serial tumor biopsies from a sorafenib-treated HCC patient". Proc. Natl. Acad. Sci. USA. 113:1381-1386 (2016) ([26])). Our results do not support this; however, there is a body of evidence that cell adhesion is related to the response of HCC to sorafenib, although the precise mechanisms involving adhesion proteins remain to be characterized.

Among this predicting protein signature in response to sorafenib, we found that TKT was the most recurrently overexpressed protein in poor responders. This result highlights TKT as a marker and as an important therapeutic target. A previous study had already associated TKT depletion with *in vitro* sensitivity of HCC cells to sorafenib treatment (Xu IM-J, et al. ([23])). We confirmed and complemented these results in patients, which emphasizes the relevance of our theranostic signature. Furthermore, TKT had never been proposed as a predictive factor for response to sorafenib treatment of HCC but we provide a strong argument from the patient analysis to strengthen this lead. In contrast, TKT did not appear to be involved in sensitivity to lenvatinib therapy, making it a good marker for choosing between the two first-line multikinase inhibitors.

At a time when dual combination therapy is a highly explored strategy for advanced HCC, the use of a TKT inhibitor should be explored. Previously, oxythiamine, an inhibitor of the PPP not specific to TKT has already been shown to be effective *in vitro* and *in vivo* in HCC cells (Xu IM-J, et al. ([23])). In our hands, we did not confirm the effect of oxythiamine on the growth of HUH7 cells. However, we have shown that TKT in hepatocytes is predominantly expressed in the cell nucleus whereas PPP is cytoplasmic. A recent study described the metabolic reprogramming in HCC, highlighting the nuclear translocation of TKT and its interaction with different kinases ans transcriptional co-regulators (Qin Z, et al ([22])). In this context, the relationship of nuclear TKT function to sorafenib sensitivity remains to be fully understood, but specific inhibition of TKT activity in the nucleus would be pharmacologically relevant and is an avenue to follow.

Once the use of TKT as a biomarker predictive of response to sorafenib is validated, quantification of TKT before treatment would already be a valuable indicator for the current choice of treatment between the two first-line multikinase inhibitors. To address it, quantification of TKT by mass spectrometry may be a first option for predicting the response to sorafenib and addressing IHC defects. We believe that analyzing the entire proteomic profile that is statistically strongest, is a promising methodological option that can be used in routine clinical practice. This analytical strategy could be extended to multikinase inhibitors currently used in first- or second-lines and for immunotherapies with a provisional authorization for first-line treatment. In addition, in the predictive signature of the sorafenib response, we identified proteins (A2M and IGHG1) involved in tumor-related immunity whose expression levels were higher in good responders. These identifications are encouraging to explore tumor proteomic profiles predicting the response to the combined anti-VEGF/anti-PDL1 immunotherapies. Moreover, thanks to laser microdissection we will also be able to more precisely target immunogenic areas more precisely to access information on the cell types involved in the response to immunotherapies.

The advantage of our process is that it is applicable on routine needle biopsies used in usual care. Our study, encourages to perform hepatic biopsies for a theranotic approach in HCC. There is still a lively debate around HCC biopsies. Pathological analysis can provide robust histoprognostic markers such as subtype of HCC, degree of differentiation, biliary or progenitor phenotype and index of proliferation. Efforts are focused on identifying non-invasive theranostic biomarkers (blood biomarkers, circulating DNA, circulating cells, exosomes). However, these different strategies entail identifying a tiny part of the tumor diluted in blood whose dynamic range is highly variable. Especially since this tiny part must also have a predictive value of the response to treatment. As non-invasive strategies are not yet available and even if biopsies represent also a small part of the total, we have easy access to large amount of information. In the future, theranostic proteomic profile identified for each treatment of advanced HCC could be used as references and statistically compared to any new patient awaiting treatment. Thus, proteomic profiling using machine learning would allow personalized management and precision medicine. Furthermore, phosphoproteomic analysis on fixed biopsies could provide access to the activation status of signaling pathways, which would provide more accurate functional information.

In conclusion, through this study, we have shown that quantification of the proteins expressed in advanced HCC allows predicting the response to treatment. This proof-of-concept encourages collecting biopsy samples in surgical practice, allows implementing precision medicine and may, ultimately, lead to a significant increase in treatments efficacy and consequently in patient's survival.

### Reference List

1. Sala M, et al.: "ASS1 Overexpression: A Hallmark of Sonic Hedgehog Hepatocellular Adenomas"; Recommendations for Clinical Practice. Hepatol. Commun. 4, 809-824 (2020).
2. Gustafsson O, et al. P. Proteomic developments in the analysis of formalin-fixed tissue. Biochim. Biophys. Acta BBA - Proteins Proteomics 1854, 559-580 (2015).
3. Coscia F, et al. A streamlined mass spectrometry-based proteomics workflow for largescale FFPE tissue analysis. J. Pathol. 251, 100-112 (2020).
4. Henriet E, et al.: "Argininosuccinate synthase 1 (ASS1): A marker of unclassified hepatocellular adenoma and high bleeding risk". Hepatol. Baltim. Md 66, 2016-2028 (2017).
5. Ahmed M, et al. Next-generation protein analysis in the pathology department. J. Clin. Pathol. 73, 1-6 (2020).
6. Jin P, et al. Pathology, proteomics and the pathway to personalised medicine. Expert Rev. Proteomics 15, 231-243 (2018).
7. Ferlay J, et al. Cancer incidence and mortality worldwide: sources, methods and major patterns in GLOBOCAN. Int. J. Cancer. 136:E359-386 (2012).
8. Di Tommaso L, et al. Role of liver biopsy in hepatocellular carcinoma. World. J Gastroenterol. 25:6041-6052 (2019).
9. Hollebecque A, et al. Systemic treatment of advanced hepatocellular carcinoma: from disillusions to new horizons. Eur J Cancer. 51:327-339 (2015).
10. Kudo M, et al. Lenvatinib versus sorafenib in first-line treatment of patients with unresectable hepatocellular carcinoma: a randomised phase 3 non-inferiority trial. Lancet. 391:1163-1173 (2018).
11. Bouyssié D, et al. Proline: an efficient and user-friendly software suite for large-scale proteomics. Bioinforma. Oxf. Engl. 36, 3148-3155 (2020).
12. Nault J-C, et al: "Molecular Classification of Hepatocellular Adenoma Associates With Risk Factors, Bleeding, and Malignant Transformation". Gastroenterology 152, 880-894.e6 (2017).
13. Zucman-Rossi J, et al.: "Genetic Landscape and Biomarkers of Hepatocellular Carcinoma". Gastroenterology 149, 1226-1239.e4 (2015).
14. Ault G.T., et al.: Selective management of hepatic adenomas. Am. Surg. 62, 825-829 (1996).
15. Marisi G, Cucchetti A, Ulivi P, et al. Ten years of sorafenib in hepatocellular carcinoma: Are there any predictive and/or prognostic markers?. World J. Gastroenterol. 24:4152-4163 (2018).
16. Pinyol R, et al. Molecular predictors of prevention of recurrence in HCC with sorafenib as adjuvant treatment and prognostic factors in the phase 3 STORM trial. Gut. 68:1065-1075 (2019).
17. Abou-Alfa GK, et al. Phase II study of sorafenib in patients with advanced hepatocellular carcinoma. J Clin Oncol. 24:4293-4300 (2006).
18. Chu JS, et al. Expression and prognostic value of VEGFR-2, PDGFR-β, and c-Met in advanced hepatocellular carcinoma. J. Exp. Clin. Cancer Res. 32:16 (2013).
19. Hagiwara S, et al. Activation of JNK and high expression level of CD133 predict a poor response to sorafenib in hepatocellular carcinoma. Br J. Cancer. 106:1997-2003 (2012).
20. Vial G, et al. Antigenic Mimicry in Paraneoplastic Immune Thrombocytopenia. Front Immunol. 10:523 (2019).
21. Li Y, et al. Transketolase contributes to hepatocellular carcinoma migration, invasion, angiogenesis, and tumorigenesis. Transl. Cancer Res. 7:1-9 (2018).
22. Qin Z, et al. Transketolase (TKT) activity and nuclear localization promote hepatocellular carcinoma in a metabolic and a non-metabolic manner. J. Exp. Clin. Cancer Res. 38:154 (2019).
23. Xu IM-J, Lai RK-H, Lin S-H, et al. Transketolase counteracts oxidative stress to drive cancer development. Proc. Natl. Acad. Sci. USA. 113:E725-734 (2016).
24. Li M, et al. The nuclear translocation of transketolase inhibits the farnesoid receptor expression by promoting the binding of HDAC3 to FXR promoter in hepatocellular carcinoma cell lines. Cell Death Dis. 11:31 (2020).
25. Cervello M, et al. Molecular mechanisms of sorafenib action in liver cancer cells. Cell Cycle. 11:2843-2855 (2012).
26. Dazert E, et al. Quantitative proteomics and phosphoproteomics on serial tumor biopsies from a sorafenib-treated HCC patient. Proc. Natl. Acad. Sci. USA. 113:1381-1386 (2016).

## Claims

1. A computer-implemented method for determining a reference proteomic profile of a pathological condition in a subject, comprising steps of :
- label free quantification of the relative protein abundances of a tissue sample from at least one subject for which a diagnosis of a pathological condition of said tissue has been previously established, said sample containing non-pathological and pathological tissue,
- determining the proteomic profile of said sample by calculating the ratio of the relative abundances, for each protein, of said protein in the pathological tissue with respect to said protein in the non-pathological tissue,
- determining a reference proteomic profile for the previously diagnosed pathological condition, by means of a statistical test established between at least two groups of subjects.

2. Method according to claim 1, wherein said sample is obtainable by macrodissection or laser microdissection of a formalin-fixed paraffin-embedded or frozen tissue.

3. Method according to claim 1 or 2, wherein the label free quantification of the relative protein abundances is realized by mass spectrometry.

4. Method according to anyone of the preceding claims, wherein the pathological condition is chosen among a cancer, an autoimmune disease, a thrombosis, an inflammatory disease, an infection, a graft or prosthesis rejection, or a benign tumor.

5. A data processing device comprising means for carrying out the method of anyone of claims 1 to 4.

6. An *ex vivo* method for analysing a tissue likely to be pathological, in a subject, comprising the following steps:
- label-free quantification of the relative protein abundances of a tissue sample from said subject, said sample containing non-pathological tissue and tissue likely to be pathological,
- determining the proteomic profile of said sample, by calculating the ratio of the relative abundances, for each protein, of the relative amount of said protein in the tissue likely to be pathological with respect to the amount of said protein in the non-pathological tissue,
- calculating the similarity score between the proteomic profile of said sample and at least one reference proteomic profile obtained by the method defined in anyone of claims 1 to 4.

7. An *ex vivo* method according to claim 6, wherein said analysis is an analysis of the profile of protein expression deregulation in said tissue likely to be pathological with respect to a reference profile.

8. An ex *vivo* method of diagnosing a pathology in a subject, comprising carrying out the ex *vivo* method of analysis as defined in claim 6 or 7, wherein a similarity score between the proteomic profile of the sample from said subject and the reference proteomic profile of a pathology is indicative of said pathology.

9. An ex *vivo* method of proteomic profiling of a tissue, comprising the following steps:
- label-free quantification of the relative protein abundances of a sample of said tissue, said sample containing non-pathological tissue and tissue likely to be pathological,
- determination of the proteomic profile of said tissue, by calculating the ratio, for each protein, of the relative amount of said protein in the tissue likely to be pathological with respect to the amount of said protein in the non-pathological tissue.

10. An *ex vivo* method for assessing a risk of transformation of a benign tumour to cancer in a subject, comprising carrying out the ex *vivo* method of analysis of a tissue according to claim 6 or 7, wherein a similarity score of the proteomic profile of the sample of said subject with the reference proteomic profile of a cancer is indicative of a risk of transformation of said benign tumour to cancer.

11. An ex *vivo* method according to claim 10, wherein said benign tumour is a hepatocellular adenoma, and said cancer is a liver cancer, in particular a hepatocellular carcinoma.

12. An ex *vivo* method for the differential diagnosis of benign and malignant tumours in a subject, comprising carrying out the ex *vivo* diagnostic method as defined in claim 8, wherein a similarity score of the proteomic profile of the sample from said subject with the reference proteomic profile of a tumour is indicative of said tumor, and optionally its subtype and malignant transformation state.

13. An ex *vivo* method according to claim 12, wherein the tumours are liver tumours.

14. An ex *vivo* method according to claim 12, wherein the benign tumours are hepatocellular adenoma and malignant tumour are hepatocellular carcinoma.

15. An ex vivo method according to claim 14, wherein the hepatocellular adenoma belongs to at least one subtype selected from H-HCA, IHCA, b-HCA, b-IHCA and sh-HCA.

16. An *ex vivo* method for identifying at least one biomarker of a pathology, comprising carrying out the method defined in anyone of claims 1 to 4.

17. An *ex vivo* method for predicting the efficacy of a treatment for a pathology in a subject, comprising carrying out the ex *vivo* diagnostic method as defined in claim 8, wherein a similarity score between the proteomic profile of the sample from said subject and the reference proteomic profile of the pathology is predictive of a response or non-response to the treatment for said pathology.

18. An ex *vivo* method according to claim 17, wherein the pathology is a liver cancer, in particular a hepatocellular carcinoma.
